# EUROPÄISCHE PATENTANMELDUNG

(11) **EP 1 146 774 A2**
(43) Veröffentlichungstag der Anmeldung: **17.10.2001**
(21) Anmeldenummer: 00119195.6
(22) Anmeldetag: 05.09.2000
(51) Int. Cl.: H04R 25/00

(54) **Teilimplantierbares System zur Rehabilitation einer Hörstörung**

(30) Priorität: 13.04.2000 DE 10018361
(71) Anmelder: IMPLEX Aktiengesellschaft Hearing Technology, 85737 Ismaning (DE)
(72) Erfinder: Leysieffer, Hans, Dr.-Ing., 82024 Taufkirchen (DE); Waldmann, Bernd, Dr., 81927 München (DE)
(74) Vertreter: Schwan, Gerhard, Dipl.-Ing.

(57) **Zusammenfassung**

Mindestens teilweise implantierbares System zur Rehabilitation einer Hörstörung, das mindestens einen Sensor (Mikrofon) (40) zur Aufnahme des Schallsignals und dessen Umwandlung in entsprechende elektrische Signale, eine elektronische Signalverarbeitungseinheit (34, 34', 62) zur Audiosignalverarbeitung und -verstärkung, eine elektrische Energieversorgungseinheit (52), welche einzelne Komponenten des Systems mit Strom versorgt, sowie eine ausgangsseitige elektromechanische Wandleranordnung (10, 22) umfasst, die aus mindestens zwei voneinander unabhängigen und örtlich getrennt angeordneten Wandlern (14, 24) zum Stimulieren des Innenohres besteht. Erfindungsgemäß sind die ausgangsseitigen elektromechanischen Wandler (14, 24) zur Anregung der flüssigkeitsgefüllten Innenohrräume des geschädigten Innenohrs ausgelegt, und die Signalverarbeitungseinheit (34, 34', 62) weist eine treibende Signalverarbeitungselektronik (42, 44) auf, die jeden der Wandler derart elektrisch ansteuert, daß auf der Basilarmembran des geschädigten Innenohres eine Wanderwellenkonfiguration entsteht, die die Art der Wanderwellenausbildung eines gesunden, nicht geschädigten Innenohres approximiert.

## Beschreibung

Die vorliegende Erfindung betrifft ein mindestens teilweise implantierbares System zur Rehabilitation einer Hörstörung, mit mindestens einem Sensor (Mikrofon) zur Aufnahme des Schallsignals und dessen Umwandlung in entsprechende elektrische Signale, einer elektronischen Anordnung zur Audiosignalverarbeitung und -verstärkung, einer elektrischen Energieversorgungseinheit, welche einzelne Komponenten des Systems mit Strom versorgt, sowie einer ausgangsseitigen elektromechanischen Wandleranordnung, die aus mindestens zwei voneinander unabhängigen und örtlich getrennt angeordneten Wandlern zum Stimulieren des Innenohres besteht.

Unter dem Begriff "Hörstörung" sollen vorliegend Innenohrschäden, kombinierte Innen- und Mittelohrschäden sowie auch zeitweise auftretende oder permanente Ohrgeräusche (Tinnitus) verstanden werden.

Die Rehabilitation sensorischer Hörstörungen mit teilimplantierbaren, elektronischen Systemen hat in den letzten Jahren einen bedeutenden Stellenwert erhalten. Insbesondere gilt dies für den Patientenkreis, bei dem das Gehör durch Unfall, Krankheit oder sonstige Einflüsse vollständig ausgefallen oder bereits von Geburt an nicht funktionsfähig ist. Ist in diesen Fällen nur das Innenohr (Cochlea) und nicht die nach zentral führende neuronale Hörbahn betroffen, kann mit elektrischen Reizsignalen der verbliebene Hörnerv stimuliert und somit ein Höreindruck erzeugt werden, der bis zu einem offenen Sprachverständnis führen kann. Bei diesen sogenannten Cochlea-Implantaten (CI) wird in die Cochlea ein Reizelektroden-Array eingeführt, das von einem elektronischen System (Elektronikmodul) angesteuert wird. Dieses Elektronikmodul ist hermetisch dicht und biokompatibel eingekapselt, und es wird operativ im knöchernen Bereich hinter dem Ohr (Mastoid) eingebettet. Das elektronische System enthält jedoch im wesentlichen nur Dekodier- und Treiberschaltungen für die Reizelektroden. Die akustische Schallaufnahme, die Wandlung dieses Schallsignals in elektrische Signale und deren weitere Verarbeitung erfolgen grundsätzlich extern in einem sogenannten Sprachprozessor, der außen am Körper getragen wird. Der Sprachprozessor setzt die vorverarbeiteten Signale entsprechend kodiert auf ein hochfrequentes Trägersignal um, das über eine induktive Kopplung durch die geschlossene Haut (transkutan) zu dem Implantat übertragen wird. Das schallaufnehmende Mikrofon befindet sich ausnahmslos außerhalb des Körpers und in den meisten Anwendungen in einem an der Ohrmuschel getragenen Gehäuse eines Hinter-dem-Ohr-Hörgerätes (HdO), und es ist mit einem Kabel mit dem Sprachprozessor verbunden. Solche Cochlea-Implantat-Systeme, deren Komponenten und Prinzipien der transkutanen Signalübertragung sind beispielhaft in folgenden Patentschriften beschrieben: US-A-5 070 535, US-A-4 4441 210, EP-A-0 200 321, US-A-5 626 629. Verfahren zur Sprachaufbereitung und -kodierung bei Cochlea-Implantaten sind beispielsweise in folgenden Patentschriften angegeben: EP-A-0 823 188, EP-A-0 190 836, US-A-5 597 380, US-A-5 271 397, US-A-5 095 904, US-A-5 601 617, US-A-5 603 726.

Neben der Rehabilitation gehörloser beziehungsweise ertaubter Patienten mit Cochlea-Implantaten existieren seit geraumer Zeit Ansätze, Patienten mit einer sensorineuralen Hörstörung, die operativ nicht behebbar ist, mit teil- beziehungsweise vollimplantierbaren Hörgeräten eine bessere Rehabilitation als mit konventionellen Hörgeräten zu bieten. Das Prinzip besteht in den überwiegenden Ausführungsformen darin, ein Ossikel des Mittelohres oder direkt das Innenohr über einen mechanischen beziehungsweise hydromechanischen Reiz zu stimulieren und nicht über das verstärkte akustische Signal eines konventionellen Hörgerätes, bei dem das verstärkte Schallsignal dem äußeren Gehörgang zugeführt wird. Der aktorische Stimulus dieser elektromechanischen Systeme wird mit verschiedenen physikalischen Wandlerprinzipien realisiert wie zum Beispiel durch elektromagnetische und piezoelektrische Systeme. Der Vorteil dieser Geräte wird hauptsächlich in der gegenüber konventionellen Hörgeräten verbesserten Klangqualität und bei vollimplantierten Systemen in der Unsichtbarkeit der Hörprothese gesehen.

Solche teil- und vollimplantierbaren elektromechanischen Hörgeräte sind beispielhaft von Yanigahara und Suzuki et al. (Arch Otolaryngol Head Neck, Surg-Vol 113, 1987, pp. 869-872; Hoke, M. (ed), Advances in Audiology, Vol. 4, Karger Basel, 1988), H.P. Zenner et al. "Erste Implantationen eines vollständig implantierbaren elektronischen Hörsystems bei Patienten mit Innenohrschwerhörigkeit", HNO 46:844-852; H. Leysieffer et al. "Ein vollständig implantierbares Hörsystem für Innenohrschwerhörige: TICA LZ 3001" HNO 46:853-863; H.P. Zenner et al. "Aktive elektronische Hörimplantate für Mittel- und Innenohrschwerhörige - eine neue Ära der Ohrchirurgie", HNO 45:749-774; H.P. Zenner et al. "Totally implantable hearing device for sensorineural hearing loss", The Lancet Vol. 352, No. 9142, Seite 1751; und in zahlreichen Patentschriften beschrieben, so unter anderem in: EP-A-0 263 254, US-A-3 557 775, US-A-3 712 962, US-A-3 764 748, US-A-5 411 467, US-A-4 352 960, US-A-4 988 333, US-A-5 015 224, US-A-5 015 225, US-A-5 277 694, US-A-5 360 388, US-A-5 772 575, US-A-5 814 095, US-A-5 951 601, US-A-5 977 689 und US-A-5 984 859. Dabei ist das Einbringen eines elektromechanischen Wandlers durch eine Eröffnung im Promontorium zur direkten Flüssigkeitsanregung im Innenohr in US-A-5 772 575, US-A-5 951 601, US-A-5 977 689 und US-A-5 984 859 beschrieben.

Viele Patienten mit einem Innenohrschaden leiden zusätzlich unter zeitweise auftretenden oder permanenten Ohrgeräuschen (Tinnitus), die operativ nicht behebbar sind und gegen die bis heute keine zugelassenen medikamentösen Behandlungsformen existieren. Daher sind sogenannte Tinnitus-Maskierer bekamt (WO-A-90/07251, EP-A-0 537 385, DE-U-296 16 956). Dies sind kleine, batteriebetriebene Geräte, die ähnlich einem Hörgerät hinter oder im Ohr getragen werden und durch artifizielle Schalle, die beispielsweise über einen Hörgeräte-Lautsprecher in den Gehörgang abgestrahlt werden, den Tinnitus auf psychoakustisch wirkende Weise verdecken ("maskieren") und das störende Ohrgeräusch so möglichst unter die Wahrnehmungsschwelle absenken. Die artifiziellen Schalle sind häufig Schmalbandgeräusche (zum Beispiel Terzrauschen), die in ihrer spektralen Lage und ihrem Lautstärkepegel über ein Programmiergerät einstellbar sind, um eine möglichst optimale Anpassung an die individuelle Ohrgeräuschsituation zu ermöglichen. Darüberhinaus existiert seit kurzem die sog. "Retraining-Methode", wobei durch die Kombination eines mentalen Trainingsprogrammes und die Darbietung eines breitbandigen Schalles (Rauschen) nahe der Ruhehörschwelle die Wahrnehmbarkeit des Tinnitus ebenfalls weitgehend unterdrückt werden soll (H. Knör, "Tinnitus-Retraining-Therapie und Hörakustik" Zeitschrift "Hörakustik" 2/97, Seiten 26 und 27). Diese Geräte werden auch als "Noiser" bezeichnet.

Bei beiden oben genannten Methoden zur apparativen Therapie des Tinnitus sind hörgeräteähnliche, technische Geräte außen am Körper im Ohrbereich sichtbar mitzuführen, die den Träger stigmatisieren und daher nicht gerne getragen werden.

In US-A-5 795 287 wird ein implantierbarer Tinnitusmaskierer mit "Direktantrieb" ("direct drive") des Mittelohres zum Beispiel über einen an die Ossikelkette angekoppelten elektromechanischen Wandler beschrieben. Dieser direkt gekoppelte Wandler kann vorzugsweise ein sogenannter "Floating Mass Transducer" (FMT) sein. Dieser FMT entspricht dem Wandler für implantierbare Hörgeräte, der in US-A-5 624 376 beschrieben ist.

In DE-C-198 58 398 und EP-A-1 014 755 werden implantierbare Systeme zur Behandlung eines Tinnitus durch Maskierung und/oder Noiserfunktionen beschrieben, bei denen der signalverarbeitende elektronische Pfad eines teil- oder vollimplantierbaren Hörsystems durch entsprechende elektronische Module so ergänzt wird, daß die zur Tinnitusmaskierung oder zur Noiserfunktion notwendigen Signale in den Signalverarbeitungsweg der Hörgerätefunktion eingespeist und die zugehörigen Signalparameter durch weitere elektronische Maßnahmen individuell an die pathologischen Bedürfnisse angepaßt werden können. Diese Anpassbarkeit kann dadurch realisiert werden, daß die notwendigen Einstelldaten der Signalerzeugungs- und Einspeiselektronik in demselben physikalischen und logischen Datenspeicherbereich des Implantatsystems hard- und softwaremäßig abgelegt beziehungsweise programmiert werden und über entsprechende elektronische Stellglieder die Einspeisung des Maskierer-beziehungsweise Noisersignals in den Audiopfad des Hörimplantats steuern.

Implantierbare Rehabilitationsgeräte der vorstehend genannten Art bestehen je nach angestrebter Funktion aus mehreren Funktionseinheiten, insbesondere einem Sensor (Mikrofon), der den einfallenden Luftschall in ein elektrisches Signal umwandelt, einer elektronischen Signalverarbeitungs-, Verstärkungs- und Implantatsteuerungseinheit, einem implantierbaren elektromechanischen beziehungsweise elektroakustischen Wandler, der die verstärkten und vorverarbeiteten Sensorsignale in mechanische beziehungsweise akustische Schwingungen umwandelt und diese über geeignete Koppelmechanismen dem geschädigten Mittel- und/oder Innenohr zuführt, oder einer cochleären Reizelektrode bei Cochlea-Implantaten, sowie einem elektrischen Energieversorgungssystem, das die genannten Module versorgt. Weiterhin kann eine externe Einheit vorgesehen sein, die dem Implantat elektrische Nachladeenergie zur Verfügung stellt, wenn die implantatseitige Energieversorgungseinheit eine nachladbare (Sekundär-) Batterie enthält. Besonders vorteilhafte Vorrichtungen und Verfahren zum Laden von nachladbaren Implantatbatterien sind in DE-A-198 38 137 sowie in US-A-5 279 292 beschrieben. Zweckmäßig ist auch eine Telemetrieeinheit vorzusehen, mit der patientenspezifische, audiologische Daten drahtlos bidirektional übertragen beziehungsweise im Implantat programmiert und damit dauerhaft gespeichert werden können, wie es von Leysieffer et al. in HNO Vol. 46, 1998, S. 853-863 beschrieben wurde.

Grundsätzlich sind bei allen genannten, mindestens teilweise implantierbaren Systemen die (Audio-) Signalverarbeitung beziehungsweise Signalerzeugung sowie die Module der Implantatsteuerung wie zum Beispiel ein geregeltes Akkumulator-Nachladesystem oder ein Telemetrie-System zur bidirektionalen Übertragung von zum Beispiel veränderlichen, patientenspezifischen Parametern implantatseitig durch fest vorgegebene Hardwareeinheiten realisiert. Dies gilt auch dann, wenn zur Signalverarbeitung beziehungsweise - erzeugung oder zum Implantatmanagement digitale Signalprozessoren oder Microcontroller beziehungsweise Mikroprozessoren eingesetzt werden, unabhängig davon, ob diese als eine sogenannte "hardwired logic", das heißt in "festverdrahteter" Logikarchitektur, realisiert sind oder ob deren Betriebsprogramme in Festwertspeicherbereichen (z. B. ROM) der entsprechenden Prozessoren abgelegt sind. Diese Programme, die zum grundlegenden Betrieb des Implantats sowie zur bestimmungsgemäßen Funktion notwendig und vorgesehen sind, werden im folgenden als Betriebsprogramm beziehungsweise als Betriebssoftware bezeichnet. Diese Betriebssoftware wird bei den bekannten Implantatsystemen während der Produktion zum Beispiel durch Maskenprogrammierung der Prozessor-Speicherbereiche in das System eingebracht, und sie ist nach der Implantation nicht mehr veränderbar.

Im Gegensatz dazu werden patientenspezifische Daten wie zum Beispiel audiologische Anpaßdaten oder auch veränderbare Implantatsystemparameter (zum Beispiel als Variable in einem der oben genannten Softwareprogramme zur Regelung einer Akkumulatornachladung) vorliegend als Betriebsparameter bezeichnet. Diese Betriebsparameter können bei bekannten vollimplantierbaren Implantatsystemen nach der Implantation transkutan, das heißt drahtlos durch die geschlossene Heut, in das Implantat übertragen und damit verändert werden.

Die oben beschriebenen mindestens teilweise implantierbaren Hörsysteme zur Rehabilitation einer Innenohrschädigung, die auf einem ausgangsseitigen elektromechanischen Wandler basieren, unterscheiden sich von herkömmlichen, konventionellen Hörgeräten im wesentlichen nur dadurch, daß der ausgangsseitige akustische Stimulus (das heißt ein verstärktes Schallsignal vor dem Trommelfell) durch einen verstärkten mechanischen Stimulus des Mittel- beziehungsweise Innenohres ersetzt wird. Der akustische Stimulus eines konventionellen Hörgerätes führt schließlich über die mechanische Anregung des Trommelfells und des sich anschließenden Mittelohres auch zu einem vibratorischen, das heißt mechanischen, Reiz des Innenohres. Bezüglich der sinnvollen Audiosignalvorverarbeitung bestehen grundlegend ähnliche beziehungsweise gleiche Anforderungen. Weiterhin wird in beiden Ausführungsformen letztendlich ausgangsseitig ein örtlich lokalisierter vibratorischer Stimulus an das geschädigte Innenohr geleitet (zum Beispiel verstärkte mechanische Schwingung des Steigbügels im ovalen Fenster des Innenohres).

Grundsätzlich besteht bei diesen heute routinemäßig eingesetzten Rehabilitationen eines Innenohrschadens durch aktive Hörsysteme (gleich ob extern akustisch oder implantiert elektromechanisch) ein gravierender Nachteil, der im folgenden zum Verständnis der vorliegenden Erfindung zusammenfassend beschrieben wird: Die überwiegende Mehrheit der Innenohrschwerhörigkeiten basiert auf einer mehr oder weniger ausgeprägten Schädigung der äußeren Haarzellen im Innenohr. Diese äußeren Haarzellen, die in einer Vielzahl im Cortischen Organ entlang der Basilarmembran angeordnet sind, bilden einen Teil des sogenannten cochleären Verstärkers, der je nach örtlicher Anregung der Basilarmembran aufgrund einer Wanderwellenausbildung diesen örtlichen Anregungsbereich bei kleinen Pegeln und damit kleinen Wanderwellenamplituden mechanisch aktiv entdämpft und somit zu einer Empfindlichkeitssteigerung führt. Diese aktive Entdämpfung beruht auf einem sehr komplexen, efferent gesteuerten Prozeß, der hier nicht näher beschrieben wird. Es wird weiterhin angenommen, daß sich bei sehr hohen Pegeln der Innenohranregung aufgrund hoher Lautstärke dieser Effekt in seiner Wirkung umkehrt und damit die Wanderwellenamplitude örtlich herabsetzt und damit aktiv bedämpft. Diese nichtlineare Kennlinie des cochleären Verstärkers, der in mehreren hundert örtlich begrenzt wirkenden Funktionseinheiten entlang des Cortischen Organs angeordnet ist, ist für die Funktion des gesunden Innenohres von maßgeblicher Bedeutung. Bei teilweisem oder vollständigem Ausfall der äußeren Haarzellen entstehen neben dem Empfindlichkeitsverlust, der zu einer Hörschwellenanhebung führt, jedoch noch weitere Nachteile: die beschriebene aktive Entdämpfung der Basilarmembran führt zu hohen Güten der Einhüllenden der Wanderwellen, die wesentlich für das Frequenzunterscheidungsvermögen (Tonhöhenunterschiede) verantwortlich sind. Fehlt diese hohe Güte durch Ausfall oder Teilschädigung der äußeren Haarzellen, kann der Betroffene deutlich schlechter Tonhöhenunterschiede wahrnehmen. Die Anhebung der Hörschwelle führt darüber hinaus zu einer Verringerung des Dynamikbereiches, da sich die obere Empfindungsgrenze (Unbehaglichkeitsschwelle) bei einer Innenohrschwerhörigkeit nicht mit anhebt. Diese Reduktion der Dynamik hat eine Versteilerung des Lautstärkeempfindens zur Folge, was als positives Recruitment bezeichnet wird. Die beschriebenen Effekte, die durch Schädigung oder Ausfall der äußeren Haarzellen hervorgerufen werden, führen in der Gesamtwirkung für den Betroffenen zur Minderung der Sprachverständlichkeit, insbesondere in störlärmerfüllter Umgebung (zusammenfassende Darstellung in Zenner, H.P.: "Hören", Georg Thieme Verlag Stuttgart, New York, 1994, Seiten 20-23, 107 und 108, und LePage, E.W., Johnstone, M.B.: "Non-linear mechanical behaviour of the basilar membrane in the basal turn of the guinea pig cochlea". Hearing Research 2 (1980), 183-189).

Wesentliche Konsequenz dieses beschriebenen Mechanismus ist, daß, wie oben angegeben, sowohl bei konventionellen, akustischen Hörgeräten wie auch bei teil- oder vollimplantierbaren Hörsystemen die wichtigen Funktionen der geschädigten äußeren Haarzellen und damit des cochleären Verstärkers nicht ersetzt oder zumindest teilweise wiederhergestellt werden können. Aus DE-C-198 40 211 ist eine Wandleranordnung für teil- oder vollimplantierbare Hörgeräte zur direkten mechanischen Anregung des Mittel-oder Innenohres bekannt, die mit einem piezoelektrischen Wandlerelement und zusätzlich mit einem elektromagnetischen Wandler versehen ist, die in einem gemeinsamen Gehäuse untergebracht sind und die beide über dasselbe Koppelelement mit einem Mittelohr-Ossikel oder direkt mit dem Innenohr koppelbar sind. Es sind ferner implantierbare Hörsysteme bekannt (US-A-5 997 466, US-A-6 005 955), die mit zwei oder mehr ausgangsseitigen elektromechanischen Wandlern in einer oder örtlich getrennten Anordnungen arbeiten; diese Ausführungsformen sind aber eindeutig dahingehend beschrieben, daß die Systemauslegung mit mehr als einem Wandler eine lineare Superposition der Auslenkungsfrequenzgänge der einzelnen Wandler ermöglicht, die im Ergebnis eine spektral möglichst optimierte beziehungsweise gezielt frequenzabhängig einstellbare oder programmierbare ausgangsseitige Anregungsform der Cochlea erlaubt und somit zu einem spektral ausgeglichenen und ausreichenden Lautstärkeeindruck des Implantatsystems führen soll. Eine Rehabilitation des cochleären Verstärkers mit den oben beschriebenen Merkmalen ist durch diese Ausführungsformen beziehungsweise beschriebenen Signalvorverarbeitungsmethoden jedoch nicht möglich.

Der Erfindung liegt die Aufgabe zugrunde, ein mindestens teilweise implantierbares System zur Rehabilitation einer Hörstörung zu schaffen, das in der Lage ist, die Funktion des cochleären Verstärkers mindestens teilweise zu ersetzen oder wiederherzustellen.

Diese Aufgabe wird dadurch gelöst, daß bei einem mindestens teilweise implantierbaren System zur Rehabilitation einer Hörstörung, das mindestens einen Sensor (Mikrofon) zur Aufnahme des Schallsignals und dessen Umwandlung in entsprechende elektrische Signale, eine elektronische Signalverarbeitungseinheit zur Audiosignalverarbeitung und -verstärkung, eine elektrische Energieversorgungseinheit, welche einzelne Komponenten des Systems mit Strom versorgt, sowie eine ausgangsseitige elektromechanische Wandleranordnung umfasst, die aus mindestens zwei voneinander unabhängigen und örtlich getrennt angeordneten Wandlern zum Stimulieren des Innenohrs besteht, erfindungsgemäß die ausgangsseitigen elektromechanischen Wandler zur Anregung der flüssigkeitsgefüllten Innenohrräume des geschädigten Innenohrs ausgelegt sind, und daß die Signalverarbeitungseinheit eine treibende Signalverarbeitungselektronik aufweist, die jeden der Wandler derart elektrisch ansteuert, daß auf der Basilarmembran des geschädigten Innenohres eine Wanderwellenkonfiguration entsteht, die die Art der Wanderwellenausbildung eines gesunden, nicht geschädigten Innenohres approximiert.

Seit kurzer Zeit ist aus CI-Implantationen wissenschaftlich bekannt, daß auch bei nicht vollständiger Taubheit Cochlea-Implantate (CIs) erfolgreich angewendet werden können, wenn mit einem konventionellen Hörgerät keine ausreichende Sprachdiskrimination mehr zu erreichen ist. Interessanterweise konnte nachgewiesen werden, daß die wesentlichen Innenohrstrukturen, die die akustische Resthörigkeit ermöglichen, zum Teil oder weitgehend langzeitstabil erhalten werden können, wenn eine CI-Elektrode in die Cochlea eingeführt wird (Ruh, S. et al.: "Cochlear Implant bei resthörigen Patienten", Laryngo-Rhino-Otol. 76 (1997), 347-350; Müller-Deile, J. et al.: "Cochlear-Implant-Versorgung bei nicht tauben Patienten?", Laryngo-Rhino-Otol. 77 (1998), 136-143; Lehnhardt, E.: "Intracochlear placement of cochlear implant electrodes in soft surgery technique", HNO 41 (1993), 356-359). Die vorliegende Erfindung baut auf diesen Erkenntnissen auf. Insbesondere wird bei einer Innenohrschwerhörigkeit, die nicht das Ausmaß einer an Taubheit grenzenden Schädigung aufweist, ein elektromechanisches Wandler-Array statt einem elektrischen Reizelektrodenarray in der Cochlea klinisch so sicher anwendbar sein, daß eine Rehabilitation des Innenohrschadens durch elektronische Simulation des cochleären Verstärkers mit besseren Resultaten möglich ist als bei konventionellen, akustischen Hörgeräten beziehungsweise implantierbaren Hörsystemen nach dem heutigen, oben beschriebenen Stand der Technik. Weiterhin wird mit dem angegebenen, mehrkanaligen Hörimplantatsystem auch ein zumindest peripher zu lokalisierender Tinnitus effektiver maskierbar sein als mit bekannten, konventionellen Tinnitus-Maskern.

Vorteilhafte weitere Ausgestaltungen der Erfindung ergeben sich aus den Unteransprüchen.

Vorzugsweise sind die ausgangsseitigen elektromechanischen Wandler für eine direkte Anregung der flüssigkeitsgefüllten Innenohrräume des geschädigten Innenohrs ausgelegt. Durch eine solche direkte Stimulation der Cochlea wird das Auftreten von Rückkopplungen, das heißt die Einkopplung des Ausgangssignals in den Sensor (Mikrofon), vermieden oder weitgehend reduziert, weil die Ossikelkette und damit das Trommelfell nicht beziehungsweise deutlich reduziert zu Schwingungen angeregt wird. Dies ist insbesondere dann von Vorteil, wenn ein Schallsensor (Mikrofonfunktion) in unmittelbarer Nähe zum Trommelfell appliziert wird, wie dies aus US-A-5 814 095 und US-A 5 999 632 bekannt ist.

Eine direkte Anregung der flüssigkeitsgefüllten Innenohrräume des geschädigten Innenohrs kann insbesondere durch ein intracochleäres Array von ausgangsseitigen elektromechanischen Wandlern erreicht werden. Ein derartiges Wandler-Array wird unmittelbar in einen flüssigkeitsgefüllten Raum des Innenohres (scala tympani oder scala vestibuli) implantiert.

Das intracochleäre Wandler-Array hat vorzugsweise einen Gesamtdurchmesser im Bereich von 0,4 mm (apikaler Bereich) bis 2,0 mm (basaler Bereich) und eine Gesamtlänge zwischen 5 mm und 50 mm. Es weist zweckmäßig einen Träger aus einem biokompatiblen und im Innenohr biostabilen Material, vorzugsweise einem Polymer, insbesondere einem Silikon, auf. Die einzelnen ausgangsseitigen elektromechanischen Wandler können dabei aus Biokompatibilitätsgründen so in den Träger eingebettet sein, daß sie von einer dünnen Schicht des Trägermaterials vollständig umgeben sind.

Um eine mechanische Wellenausbreitung von einem Wandler innerhalb des Trägers zu benachbarten Wandlern zu minimieren, sind in weiterer Ausgestaltung der Erfindung zwischen den einzelnen ausgangsseitigen elektromechanischen Wandlern mechanische Dämpfungselemente in den Träger eingebettet, wobei das Material der Dämpfungselemente bei ähnlicher Querschnittsgeometrie wie die des Trägers vorzugsweise so gewählt ist, daß zur Erzielung hoher Dämpfungswerte ein großer mechanischer Impedanzunterschied zu dem Trägermaterial besteht.

Das intracochleäre Wandler-Array oder Teile desselben (insbesondere die Wandler und/oder die Dämpfungselemente) kann beziehungsweise können mikrosystemtechnisch hergestellt werden.

Entsprechend einer abgewandelten Ausführungsform der Erfindung, ist ein extracochleäres mehrkanaliges Array von ausgangsseitigen elektromechanischen Wandlern vorgesehen, das von außen auf der Cochlea fixiert wird.

Ein solches extracochleäres Wandler-Array kann als Ganzes einfach und mit hoher Genauigkeit in aus der Halbleiterfertigung üblichen Verfahren mikrosystemtechnisch, zum Beispiel fotolithographisch, entwickelt und hergestellt werden. Solche Verfahren gestatten eine hohe Miniaturisierung und eine exzellente Reproduzierbarkeit der einzelnen Wandler auf einem Array. Die Eigenschaften der mikrosystemtechnischen Erzeugung sind vorliegend besonders vorteilhaft, weil es bei der beabsichtigten Funktion des Arrays sehr auf einen Phasengleichlauf der einzelnen Wandler auf dem Array ankommt. Einzelheiten von mikrosystemtechnischen Verfahren sind unter anderem in WO-A-99/03146 beschrieben und bedürfen vorliegend keiner näheren Erläuterung.

Für das extracochleäre Wandler-Array kann vorteilhaft ein Substrat vorgesehen sein, das ein elektrisches Anschlußfeld enthält, das mikrosystemtechnisch mitgefertigt und für den Anschluß einer mehrpoligen, biokompatiblen Implantatleitung zu einem Modul ausgelegt ist, das die treibende Signalverarbeitungselektronik enthält. Das Substrat des extracochleären Wandler-Arrays kann ferner mit einem mikrosystemtechnisch mitgefertigten Elektronikmodul versehen sein, das insbesondere Treiberstufen zum Ansteuern der ausgangsseitigen elektromechanischen Wandler und/oder eine Dekodierlogik und Umsetzerbausteine für den Anschluß einer polreduzierten Implantatleitung enthalten kann. So kann der Array-Anschluß aus nur drei Leitungen bestehen, und zwar insbesondere einer Masseleitung, einer Datenleitung und einer Taktsignalleitung, wobei die Versorgung mit elektrischer Betriebsenergie durch Phantomspeisung auf der Taktsignalleitung erfolgen kann.

Das Elektronikmodul kann des weiteren einen Schnittstellenbaustein für eine digitale Datenübertragung über die Implantatleitung, vorzugsweise mittels einer Lichtleitfaser, und/oder bei serieller Datenübertragung auf der Implantatzuleitung den Wandlern zugeordnete D/A-Wandler und Treiberbausteine enthalten.

Zweckmäßig ist das extracochleäre Wandler-Array einschließlich Trägeraufbau (Substrat) mit einer biokompatiblen Ummantelung ausgestattet, die vorzugsweise aus von der Implantattechnologie bekannten Polymeren, insbesondere Polytetrafluorethylen, Polyurethan, oder Silikonen, besteht.

Für eine direkte Anregung der flüssigkeitsgefüllten Innenohrräume kann bei Verwendung eines extracochleären Wandler-Arrays dadurch gesorgt werden, daß die Wandler jeweils ein ausgangsseitiges Koppelelement aufweisen, das so ausgebildet ist, daß es durch einen artifiziellen Zugang zum Innenohr (Eröffnungen beziehungsweise Bohrungen in der knöchernen Außenwand der Cochlea, sog. "Cochleostomien") hindurchragt.

Die ausgangsseitigen elektromechanischen Wandler sind bevorzugt hermetisch dicht aufgebaut, und sie können grundsätzlich nach jedem bekannten elektromechanischen Wandlerprinzip arbeiten und insbesondere als elektromagnetische, elektrodynamische, piezoelektrische, magnetostriktive oder dielektrische (kapazitive) Wandler ausgebildet sein. Insbesondere für eine extracochleäre Array-Ausführungsform sind das piezoelektrische Prinzip und das dielektrische beziehungsweise kapazitive Prinzip besonders bevorzugt. Bei Anwendung des piezoelektrischen Wandlerprinzips sind sie vorteilhaft unter Verwendung von PZT-Keramik (Blei-Zirkonat-Titanat) oder von PVDF (Polyvinylidenfluorid) aufgebaut. Insbesondere sind die ausgangsseitigen elektromechanischen Wandler, vorzugsweise mit Ausnützung von geometrischen Gestalttransformationen, insbesondere des Bimorph-Prinzips, des Unimorph-Prinzips oder des Heteromorph-Prinzips mit passiven Materialpartnern, zweckmäßig so ausgeführt, daß sie bei gegebener Wandlerspannung eine maximale Auslenkung bei minimaler elektrischer Leistungsaufnahme erzeugen.

In weiterer Ausgestaltung der Erfindung sind die ausgangsseitigen elektromechanischen Wandler in dem betreffenden Wandler-Array äquidistant oder in logarithmischen Distanzen - entsprechend der tonotopen Frequenz-Orts-Zuordnung längs der Basilarmembran des Innenohres - verteilt angeordnet, wobei im Falle einer tonotopen Anordnung eine Wandleranzahl von 20 bis 24 gemäß den psychoakustischen Frequenzgruppen zu besonders günstigen Ergebnissen führen kann.

Die ausgangsseitigen elektromechanischen Wandler haben zweckmäßig einen Übertragungsbereich von etwa 100 Hz bis etwa 10 kHz, und sie sind vorzugsweise hoch abgestimmt, das heißt, ihre erste mechanische Resonsanzfrequenz liegt am oberen Ende des angestrebten Übertragungsfrequenzbereiches, insbesondere bei etwa 8 kHz bis etwa 10 kHz. Dadurch wird erreicht, daß der Auslenkungsfrequenzgang der Wandler im Übertragungsbereich weitgehend frei ist von Resonanzen und bei Spannungseinprägung und Verwendung piezoelektrischer Wandler frequenzunabhängig eben. Im Übertragungsbereich tritt somit keine Welligkeit auf.

Die Signalverarbeitungseinheit weist zweckmäßig eine Vorverarbeitungsanordnung zum Vorverstärken und/oder Filtern sowie zum Analog-Digital- (A/D-) Wandeln der Schallsensorsignale auf. Sie kann insbesondere einen Antialiasing-Filter umfassen. Bei Vorhandensein mehrerer Schallsensoren ist vorzugsweise jedem der Schallsensoren ein eigener Analog-Digital-Wandler nachgeschaltet.

In weiterer Ausgestaltung der Erfindung kann die Signalverarbeitungseinheit Softwaremodule enthalten, die parallel zum Hörgerätebetrieb die Maskierung eines Tinnitus ermöglichen. Mit dem vorliegenden mehrkanaligen Hörimplantatsystem kann ein zumindest peripher zu lokalisierender Tinnitus effektiver maskiert werden als mit bekannten, konventionellen Tinnitus-Maskern.

Die Signalverarbeitungseinheit weist vorteilhaft einen digitalen Signalprozessor zum Verarbeiten der A/D-gewandelten und gegebenenfalls mittels der Vorverarbeitungsanordnung vorverarbeiteten Schallsensorsignale und/oder zum Generieren von digitalen Signalen für eine Tinnitusmaskierung auf, wobei der ausgangsseitigen elektromechanischen Wandleranordnung mindestens ein Digital-Analog-Wandler zugeordnet ist und vorzugsweise den ausgangsseitigen elektromechanischen Wandlern jeweils ein eigener Digital-Analog-Wandler vorgeschaltet ist.

In weiterer Ausgestaltung der Erfindung enthält die Signalverarbeitungselektronik Softwaremodule, welche die Ansteuerung der ausgangsseitigen elektromechanischen Wandler so vornehmen, daß die spektralen, zeitlichen, amplituden- und phasenbezogenen Wandlersignaleigenschaften derart bemessen sind, daß eine Wanderwelle auf der Basilarmembran des geschädigten Innenohres erzeugt wird, die der eines gesunden Gehörs möglichst nahe kommt.

Die Softwaremodule können dabei statisch in der Weise ausgelegt sein, daß sie aufgrund wissenschaftlicher Erkenntnisse einmalig in einem Programmspeicher des digitalen Signalprozessors abgelegt werden und unverändert bleiben. Liegen dann aber später zum Beispiel aufgrund neuerer wissenschaftlicher Erkenntnisse verbesserte Algorithmen zur Sprachsignalaufbereitung und -verarbeitung vor und sollen diese genutzt werden, muß durch einen invasiven, operativen Patienteneingriff das gesamte Implantat oder das Implantatmodul, das die entsprechende Signalverarbeitungseinheit enthält, gegen ein neues mit der veränderten Betriebssoftware ausgetauscht werden. Dieser Eingriff birgt erneute medizinische Risiken für den Patienten und ist mit hohem Aufwand verbunden.

Diesem Problem kann dadurch begegnet werden, daß in weiterer Ausgestaltung der Erfindung eine drahtlose, bevorzugt PC-basierte, Telemetrieeinrichtung zur Übertragung von Daten zwischen dem implantierten Teil des Systems und einer externen Einheit, insbesondere einem externen Programmiersystem vorgesehen ist, wobei vorzugsweise dem Signalprozessor zur Aufnahme und Wiedergabe eines Betriebsprogramms eine wiederholt beschreibbare, implantierbare Speicheranordnung zugeordnet ist und mindestens Teile des Betriebsprogramms durch von der externen Einheit über die Telemetrieeinrichtung übermittelte Daten geändert oder ausgetauscht werden können. Auf diese Weise läßt sich nach Implantation des implantierbaren Systems die Betriebssoftware als solche verändern oder auch vollständig austauschen, wie dies für im übrigen bekannte Systeme zur Rehabilitation von Hörstörungen in der älteren EPPatentanmeldung 99 115 256.2 erläutert ist.

Bevorzugt ist die Auslegung so beschaffen, daß darüberhinaus bei vollimplantierbaren Systemen auch in an sich bekannter Weise Betriebsparameter, das heißt patientenspezifische Daten, wie beispielsweise audiologische Anpaßdaten, oder veränderbare Implantatsystemparameter (zum Beispiel als Variable in einem Softwareprogramm zur Regelung einer Batterienachladung) nach der Implantation transkutan, das heißt drahtlos durch die geschlossene Haut, in das Implantat übertragen und damit verändert werden können. Dabei sind die Softwaremodule bevorzugt dynamisch, oder mit anderen Worten lernfähig ausgelegt, um der Ausbildung einer Wanderwellenkonfiguration, welche die Art der Wanderwellenausbildung eines gesunden, nicht geschädigten Innenohres approximiert, möglichst optimal nahe zu kommen. Insbesondere können die Softwaremodule adaptiv ausgelegt sein, und eine Parameteranpassung kann durch "Training" durch den Implantatträger und weitere Hilfsmittel vorgenommen werden.

Weiterhin kann die Signalverarbeitungselektronik ein Softwaremodul enthalten, das eine möglichst optimale Simulation eines "gesunden" cochleären Verstärkers auf der Basis eines lernfähigen neuronalen Netzwerkes erreicht. Das Training dieses neuronalen Netzwerks kann wieder durch den Implantatträger erfolgen und/oder unter Zuhilfenahme weiterer externer Hilfsmittel. Insbesondere kann in dem neuronalen Netzwerk zur Simulation eines "gesunden" cochleären Verstärkers das Prinzip der "Time-Reversed Acoustics" (TRA) implementiert sein, und die Ansteuerung der ausgangsseitigen elektromechanischen Wandler kann mittels TRA so erfolgen, daß örtlich begrenzte Bereiche der Cochlea mechanisch stimuliert werden.

Die Speicheranordnung zum Speichern von Betriebsparametern und die Speicheranordnung zur Aufnahme und Wiedergabe des Betriebsprogramms können als voneinander unabhängige Speicher implementiert sein; es kann sich jedoch auch um einen einzigen Speicher handeln, in dem sowohl Betriebsparameter als auch Betriebsprogramme abgelegt werden können.

Die vorliegende Lösung erlaubt eine Anpassung des Systems an Gegebenheiten, die erst nach Implantation des implantierbaren Systems erfaßbar sind. So sind beispielsweise bei einem mindestens teilweise implantierbaren Hörsystem zur Rehabilitation einer monauralen oder binauralen Innenohrstörung sowie eines Tinnitus mit mechanischer Stimulation des Innenohres die sensorischen (Schallsensor beziehungsweise Mikrofon) und aktorischen (Ausgangsstimulator) biologischen Schnittstellen immer abhängig von den anatomischen, biologischen und neurophysiologischen Gegebenheiten, zum Beispiel von dem interindividuellen Einheilprozeß. Diese Schnittstellenparameter können individuell insbesondere auch zeitvariant sein. So können beispielsweise das Übertragungsverhalten eines implantierten Mikrofons aufgrund von Gewebebelägen und das Übertragungsverhalten eines an das Innenohr angekoppelten elektromechanischen Wandlers aufgrund unterschiedlicher Ankopplungsqualität interindividuell und individuell variieren. Solche Unterschiede der Schnittstellenparameter, die sich bei den aus dem Stand der Technik bekannten Vorrichtungen nicht einmal durch den Austausch des Implantats mindern beziehungsweise eliminieren ließen, können vorliegend durch Veränderung beziehungsweise Verbesserung der Signalverarbeitung des Implantats optimiert werden.

Bei einem mindestens teilweise implantierbaren Hörsystem kann es sinnvoll oder notwendig werden, nach Implantation verbesserte Signalverarbeitungsalgorithmen zu implementieren. Dabei sind insbesondere zu nennen:
- Sprachanalyseverfahren (zum Beispiel Optimierung einer Fast-Fourier-Transformation (FFT)),
- statische oder adaptive Störschallerkennungsverfahren,
- statische oder adaptive Störschallunterdrückungsverfahren,
- Verfahren zur Optimierung des systeminternen Signal-Rauschabstandes,
- optimierte Signalverarbeitungsstrategien bei progredienter Hörstörung,
- ausgangspegelbegrenzende Verfahren zum Schutz des Patienten bei Implantatfehlfunktionen beziehungsweise externen Fehlprogrammierungen,
- Verfahren zur Vorverarbeitung mehrerer Sensor(Mikrofon)signale, insbesondere bei binauraler Positionierung der Sensoren,
- Verfahren zur binauralen Verarbeitung zweier oder mehrerer Sensorsignale bei binauraler Sensorpositionierung zum Beispiel Optimierung des räumlichen Hörens beziehungsweise Raumorientierung,
- Phasen- beziehungsweise Gruppenlaufzeit-Optimierung bei binauraler Signalverarbeitung,
- Verfahren zur optimierten Ansteuerung der Ausgangsstimulatoren, insbesondere bei binauraler Positionierung der Stimulatoren.

Mit dem vorliegenden System lassen sich auch nach der Implantation unter anderem die folgenden Signalverarbeitungsalgorithmen implementieren:
- Verfahren zur Rückkopplungsunterdrückung beziehungsweise -minderung,
- Verfahren zur Optimierung des Betriebsverhaltens des beziehungsweise der Ausgangswandler (zum Beispiel Frequenz- und Phasengangoptimierung, Verbesserung des Impulsübertragungsverhaltens),
- Sprachsignal-Kompressionsverfahren bei Innenohrschwerhörigkeiten,
- Signalverarbeitungsmethoden zur Recruitment-Kompensation bei Innenohrschwerhörigkeiten.

Des weiteren ist bei Implantatsystemen mit einer sekundären Energieversorgungseinheit, das heißt einem nachladbaren Akkumulatorsystem, aber auch bei Systemen mit primärer Batterieversorgung davon auszugehen, daß diese elektrischen Energiespeicher mit voranschreitender Technologie immer größere Lebensdauern und damit steigende Verweilzeiten im Patienten ermöglichen. Es ist davon auszugehen, daß die Grundlagen- und Applikationsforschung für Signalverarbeitungsalgorithmen schnelle Fortschritte macht. Die Notwendigkeit oder der Patientenwunsch einer Betriebssoftwareanpassung beziehungsweise -veränderung wird daher voraussichtlich vor Ablauf der Lebensdauer der implantatinternen Energiequelle eintreten. Das vorliegend beschriebene System erlaubt eine derartige Anpassung der Betriebsprogramme des Implantats auch im bereits implantierten Zustand.

Vorzugsweise ist ferner eine Zwischenspeicheranordnung vorgesehen, in welcher von der externen Einheit über die Telemetrieeinrichtung übermittelte Daten vor dem Weiterleiten an den Signalprozessor zwischengespeichert werden können. Auf diese Weise läßt sich der Übertragungsvorgang von der externen Einheit zu dem implantierten System abschließen, bevor die über die Telemetrieeinrichtung übermittelten Daten an den Signalprozessor weitergeleitet werden.

Des weiteren kann eine Überprüfungslogik vorgesehen sein, die in der Zwischenspeicheranordnung gespeicherte Daten vor dem Weiterleiten an den Signalprozessor einer Überprüfung unterzieht. Es kann ein Mikroprozessorbaustein, insbesondere ein Microcontroller, zum implantatinternen Steuern der A/D-Wandler und/oder der D/A-Wandler und/oder des Signalprozessors über einen Datenbus vorgesehen sein, wobei zweckmäßig die Überprüfungslogik und die Zwischenspeicheranordnung in dem Mikroprozessorbaustein implementiert sind und wobei über den Datenbus und die Telemetrieeinrichtung auch Programmteile oder ganze Softwaremodule zwischen der Außenwelt, dem Mikroprozessorbaustein und dem Signalprozessor übermittelt werden können.

Dem Mikroprozessorbaustein ist vorzugsweise eine implantierbare Speicheranordnung zum Speichern eines Arbeitsprogramms für den Mikroprozessorbaustein zugeordnet, und mindestens Teile des Arbeitsprogramms für den Mikroprozessorbaustein können durch von der externen Einheit über die Telemetrieeinrichtung übermittelte Daten geändert oder ausgetauscht werden.

In weiterer Ausgestaltung der Erfindung können mindestens zwei Speicherbereiche zur Aufnahme und Wiedergabe mindestens des Betriebsprogramms des Signalprozessors vorgesehen sein. Dies trägt zur Fehlersicherheit des Systems bei, indem durch das mehrfache Vorhandensein des Speicherbereichs, welcher das beziehungsweise die Betriebsprogramme enthält, beispielsweise nach einer Übertragung von extern oder aber beim Einschalten des Implantats eine Überprüfung der Fehlerfreiheit der Software durchgeführt werden kann.

Analog hierzu kann auch die Zwischenspeicheranordnung mindestens zwei Speicherbereiche zur Aufnahme und Wiedergabe von von der externen Einheit über die Telemetrieeinrichtung übermittelten Daten aufweisen, so daß nach einer Datenübertragung von der externen Einheit noch im Bereich des Zwischenspeichers eine Überprüfung der Fehlerfreiheit der übermittelten Daten vorgenommen werden kann. Die Speicherbereiche können zur beispielsweise komplementären Ablage der von der externen Einheit übermittelten Daten ausgelegt sein. Mindestens einer der Speicherbereiche der Zwischenspeicheranordnung kann aber auch zur Aufnahme nur eines Teils der von der externen Einheit übermittelten Daten ausgelegt sein, wobei in diesem Fall die Überprüfung der Fehlerfreiheit der übermittelten Daten abschnittsweise erfolgt.

Um zu gewährleisten, daß bei Übertragungsfehlern ein erneuter Übertragungsvorgang gestartet werden kann, kann dem Signalprozessor ferner ein vorprogrammierter, nicht überschreibbarer Festspeicherbereich zugeordnet sein, in welchem die für einen "Minimalbetrieb" des Systems erforderlichen Anweisungen und Parameter gespeichert sind, beispielsweise Anweisungen, die nach einem "Systemabsturz" zumindest einen fehlerfreien Betrieb der Telemetrieeinrichtung zum Empfang eines Betriebsprogramms sowie Anweisungen zum Einspeichern desselben in die Steuerlogik gewährleisten.

Wie bereits erwähnt, ist die Telemetrieeinrichtung in vorteilhafter Weise außer zum Empfang von Betriebsprogrammen von der externen Einheit auch zur Übermittlung von Betriebsparametern zwischen dem implantierbaren Teil des Systems und der externen Einheit ausgelegt, so daß einerseits solche Parameter von einem Arzt, einem Hörgeräteakustiker oder dem Träger des Systems selbst eingestellt werden können (zum Beispiel Lautstärke), andererseits das System aber auch Parameter an die externe Einheit übermitteln kann, beispielsweise um den Status des Systems zu überprüfen.

Ein vollständig implantierbares Hörsystem der vorliegend erläuterten Art kann implantatseitig neben der aktorischen Stimulationsanordnung und der Signalverarbeitungseinheit mindestens einen implantierbaren Schallsensor und ein nachladbares elektrisches Speicherelement aufweisen, wobei in einem solchen Fall eine drahtlose, transkutane Ladevorrichtung zum Laden des Speicherelements vorgesehen sein kann. Es versteht sich jedoch, daß zur Energieversorgung auch eine Primärzelle oder eine andere Energieversorgungseinheit vorhanden sein kann, die keine transkutane Nachladung benötigt. Dies gilt insbesondere, wenn man berücksichtigt, daß in naher Zukunft vor allem durch Weiterentwicklung der Prozessortechnologie mit wesentlicher Verminderung des Energiebedarfs für elektronische Signalverarbeitung zu rechnen ist, so daß für implantierbare Hörsysteme neue Energieversorgungsformen praktisch anwendbar werden, zum Beispiel eine den See-beck-Effekt nutzende Energieversorgung, wie sie in DE-C198 27 898 beschrieben ist. Vorzugsweise ist auch eine drahtlose Fernbedienung zur Steuerung der Implantatfunktionen durch den Implantatträger vorhanden.

Bei teilimplantierbarer Ausbildung des Hörsystems sind mindestens ein Schallsensor, die elektronische Anordnung zur Audiosignalverarbeitung und -verstärkung, die Energieversorgungseinheit sowie eine Modulator/Sender-Einheit in einem extern am Körper, vorzugsweise am Kopf über dem Implantat zu tragenden externen Modul enthalten. Das Implantat weist ein intra- oder extracochleäres Wandler-Array auf, ist aber energetisch passiv und empfängt seine Betriebsenergie und Wandlersteuerdaten über die Modulator/Sender-Einheit im externen Modul.

Das beschriebene System kann monaural oder binaural ausgelegt sein. Ein binaurales System zur Rehabilitation einer Hörstörung beider Ohren weist zwei Systemeinheiten auf, die jeweils einem der beiden Ohren zugeordnet sind. Dabei können die beiden Systemeinheiten einander im wesentlichen gleich sein. Es kann aber auch die eine Systemeinheit als Master-Einheit und die andere Systemeinheit als von der Master-Einheit gesteuerte Slave-Einheit ausgelegt sein. Die Signalverarbeitungsmodule der beiden Systemeinheiten können auf beliebige Weise, insbesondere über eine drahtgebundene implantierbare Leitungsverbindung oder über eine drahtlose Verbindung, vorzugsweise eine bidirektionale Hochfrequenzstrecke, eine körperschallgekoppelte Ultraschallstrecke oder eine die elektrische Leitfähigkeit des Gewebes des Implantatträgers ausnutzende Datenübertragungsstrecke, so miteinander kommunizieren, daß in beiden Systemeinheiten eine optimierte binaurale Signalverarbeitung und Wandler-Array-Ansteuerung erreicht wird.

Die elektromechanischen Wandler können als Hohlkörper ausgebildet sein, die bei Anlegen eines Spannungssignals eine dynamische Volumenänderung erfahren und aus denen dabei in dem Wandlerhohlraum befindliche intracochleäre Flüssigkeit herausgepreßt wird, und die so ausgebildeten elektromechanischen Wandler können in einem schlauchförmigen Träger untergebracht sein, der benachbart von mindestens einem Ende der Wandler mit mindestens einer Öffnung für den Durchtritt von intracochleärer Flüssigkeit versehen ist.

Bevorzugte Ausführungsformen des erfindungsgemäßen Systems sind nachstehend unter Bezugnahme auf die beiliegenden Zeichnungen näher beschrieben. Es zeigen:
- Fig. 1: schematisch ein Ausführungsbeispiel eines intracochleären, elektromechanischen Wandler-Arrays,
- Fig. 2: ein Detail der Fig. 1,
- Fig. 3: schematisch ein Ausführungsbeispiel eines extracochleär zu applizierenden elek- tromechanischen Wandler-Arrays,
- Fig. 4: schematisch ein Ausführungsbeispiel für den Aufbau des extracochleären elek- tromechanischen Wandler-Arrays gemäß Fig. 3,
- Fig. 5: schematisch ein Ausführungsbeispiel für den Aufbau eines signalverarbeitenden Elektronikmoduls eines mindestens teilweise implantierbaren Hörsystems,
- Fig. 6: schematisch ein Ausführungsbeispiel für den Aufbau eines vollständig implan- tierbaren Hörsystems mit einem intracochleären Wandler-Array gemäß Fig. 1,
- Fig. 7: schematisch ein Ausführungsbeispiel für den Aufbau eines vollständig implan- tierbaren Hörsystems mit einem extracochleären Wandler-Array gemäß Fig. 4,

- Fig. 8: schematisch ein Ausführungsbeispiel für den Aufbau eines teilimplantierbaren Hörsystems mit einem intracochleären Wandler-Array gemäß Fig. 1,
- Fig. 9: eine binaurale Anwendung eines Hörimplantates gemäß Fig. 6, bei dem Signal- verarbeitungsmodule über eine drahtgebundene implantierbare Leitungsverbin- dung miteinander kommunizieren,
- Fig. 10: eine binaurale Anwendung eines Hörimplantates gemäß Fig. 6, bei dem die Signalverarbeitungsmodule über eine drahtlose Verbindung miteinander kom- munizieren,
- Fig. 11: eine binaurale Anwendung eines Hörimplantates gemäß Fig. 6, bei dem die Signalverarbeitungsmodule über eine körperschallgekoppelte Ultraschallstrecke miteinander kommunizieren,
- Fig. 12: eine binaurale Anwendung eines Hörimplantates gemäß Fig. 6, bei dem die Signalverarbeitungsmodule über eine das Gewebe des Implantatträgers einschließende Übertragungsstrecke miteinander kommunizieren, sowie
- Fig. 13: ein Ausführungsbeispiel eines vorliegend geeigneten elektromechanischen Wandlers.

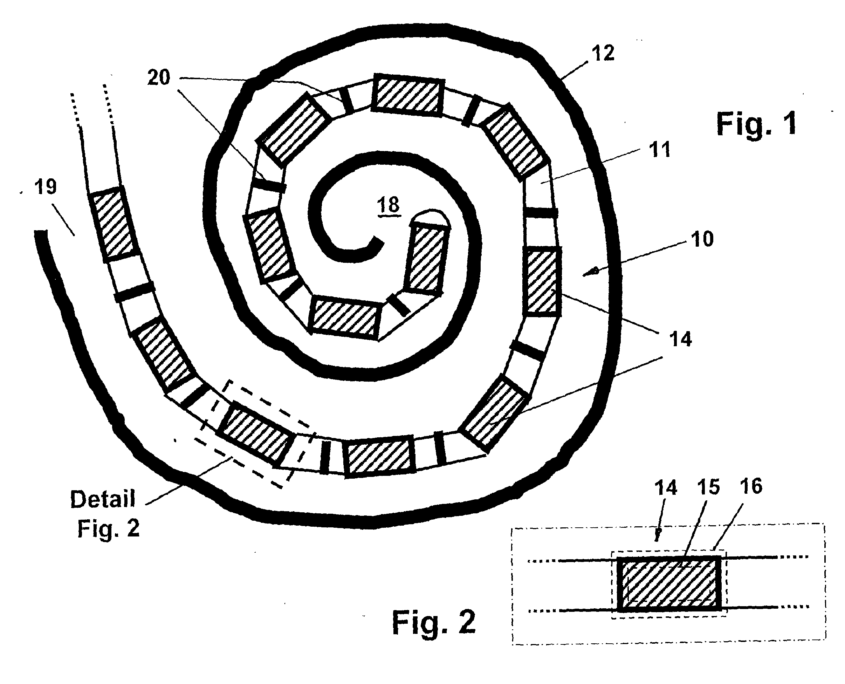

Das in Fig. 1 dargestellte, insgesamt mit 10 bezeichnete intracochleäre, elektromechanische Wandler-Array ist ähnlich einer mehrkanaligen, intracochleären Cochlea-Implantat-Elektrodenanordnung aufgebaut. Es weist einen mechanischen Träger 11 auf, der bevorzugt im wesentlichen aus einem flexiblen Formteil von vorzugsweise kreisförmigem Querschnitt besteht. Das Formteil wird durch eine artifizielle Eröffnung der Cochlea 12 oder das runde Fenster in das Innenohr geschoben. Statt einer Verteilung von elektrischen Reizelektroden einer Cochlea-Implantat-Elektrodenanordnung längs dieses Trägers 11 sind vorliegend mehrere ausgangsseitige elektromechanische Wandler 14 angeordnet, die in Fig. 1 schematisch als zylinderförmige Elemente mit ebenfalls kreisförmigem Querschnitt gezeigt sind. Innerhalb des Trägers 11 befinden sich elektrische Zuleitungen zu den Wandlern 14; diese Zuleitungen sind nicht dargestellt.

Die Wandler 14 arbeiten vorzugsweise nach dem Prinzip der dynamischen Volumenänderung aufgrund einer dynamischen Oberflächenvergrößerung beziehungsweise -verkleinerung entsprechend einem ansteuernden elektrischen Wandlerwechselspannungssignal. Eine solche dynamische Volumen- beziehungsweise Oberflächenveränderung ist schematisch in Fig. 2 dargestellt. Dort sind mit gestrichelten Linien 15 und 16 ein minimales beziehungsweise ein maximales Volumen angedeutet. Die erforderlichen Volumenveränderungen für einen adäquaten, äquivalenten Schalldruckpegel von ca. 100 dB SPL ergeben sich zu etwa 2 • 10⁻⁴ Mikroliter (US-A-5 772 575).

Die Wandler 14 sind beispielsweise äquidistant längs des Trägers 11 verteilt oder in logarithmischen Distanzen entsprechend der tonotopen Orts-Frequenz-Zuordnung längs der Basilarmembran des Innenohres. Der Gesamtdurchmesser der Wandler-Array-Anordnung 10 liegt vorzugsweise im Bereich von 0,4 mm (apikaler Bereich 18 gemäß Fig. 1) bis 2,0 mm (basaler Bereich 19 gemäß Fig. 1). Die Gesamtlänge des WandlerArrays 10 liegt zweckmäßig zwischen 5 mm und 50 mm. Die Wandlerelemente 14 sind aus Biokompatibilitätsgründen vorzugsweise so in den Träger 11 eingebettet, daß sie vollständig von einer dünnen Schicht des Trägermaterials umgeben sind (in Fig. 1 nicht dargestellt). Der Träger 11 des Wandler-Arrays 10 besteht aus einem biokompatiblen und im Innenohr biostabilen Material, vorzugsweise Polymere wie entsprechende Silikone. Zwischen den einzelnen Wandlern 14 können mechanische Dämpfungselemente 20 in den Träger 11 eingebettet sein, die die mechanische Wellenausbreitung innerhalb des Trägers zu den benachbarten Wandlerelementen minimieren. Um hohe Dämpfungswerte zu erhalten, ist vorzugsweise das Material dieser Dämpfungselemente 20 bei ähnlicher Querschnittsgeometrie wie die des Trägers 11 so gewählt, daß ein großer mechanischer Impedanzunterschied zu dem Trägermaterial besteht.

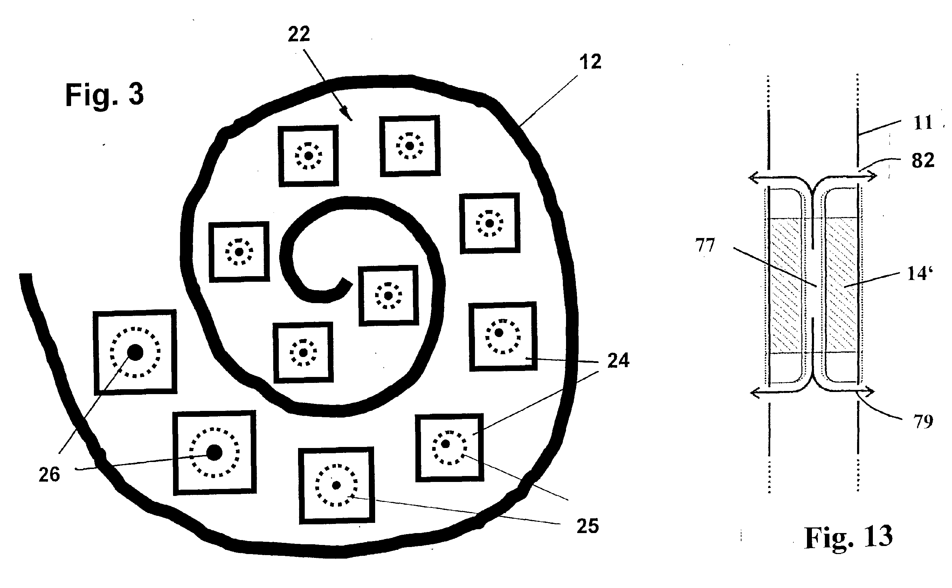

Fig. 3 zeigt schematisch ein extracochleär zu applizierendes elektromechanisches Wandler-Array 22. Dabei sind mehrere miniaturisierte ausgangsseitige elektromechanische Wandler 24 so über artifiziell in die die Cochlea 12 begrenzende knöcherne Wandung eingebrachte Öffnungen beziehungsweise Bohrungen (Cochleostomien) 25 plaziert, daß wandlerausgangsseitig angebrachte Koppelelemente 26 durch die cochleären Eröffnungen 25 in die lymphatischen Innenohrräume ragen. Mechanischen Stimuli der Wandler 24 werden intracochleär als Volumenverschiebungen erzeugt, die zu einem Höreindruck führen. Der mit den Bohrungen 25 zu versehende knöcherne Cochlea-Begrenzungsbereich ist vom Mastoid aus im Areal des Promontoriums operativ zugänglich.

Die Wandler 14 und 24 können nach jedem bekannten elektromechanischen Wandlerprinzip arbeiten, nämlich elektromagnetisch, elektrodynamisch, piezoelektrisch, magnetostriktiv oder dielektrisch (kapazitiv). Bevorzugt werden insbesondere bei der extracochleären Array-Ausfiührungsform das piezoelektrische Prinzip und das dielektrische beziehungsweise kapazitive Wandlungsprinzip. Bevorzugt wird das extracochleäre Wandler-Array 22, gegebenenfalls aber mindestens teilweise auch das intracochleäre Wandler-Array 10, mit mikrosystemtechnischen Methoden entwickelt beziehungsweise hergestellt, die eine hohe Miniaturisierung und eine exzellente Reproduzierbarkeit der einzelnen Wandler 24 auf einem Array gestatten. Diese Eigenschaften der mikrosystemtechnischen Erzeugung können besonders vorteilhaft sein, weil es erwartungsgemäß bei der beabsichtigten Funktion des Arrays sehr auf einen Phasengleichlauf der einzelnen Wandler ankommt. Ein möglicher, mikrosystemtechnischer Aufbau eines einzelnen Wandlers ist in der Patentliteratur von Ron Maynard angegeben (WO-A-99/03146).

Die einzelnen elektromechanischen Wandler 14 beziehungsweise 24 werden von einem nachstehend näher erläuterten elektronischen Vorverarbeitungssystem so angesteuert, daß durch jeweilige Wahl des spektralen Übertragungsbereiches je Wandler, der vibratorischen Amplitude und der Phasenlage der Wandler zueinander im aktorischen Gesamtergebnis der Innenohrstimulation eine Wanderwellenausbildung auf der Basilarmembran entsteht, die bei dem jeweiligen externen Schallereignis der Wanderwellenform möglichst nahekommt, die sich bei nicht geschädigtem cochleärem Verstärker und damit intakten äußeren Haarzellen ergeben würde.

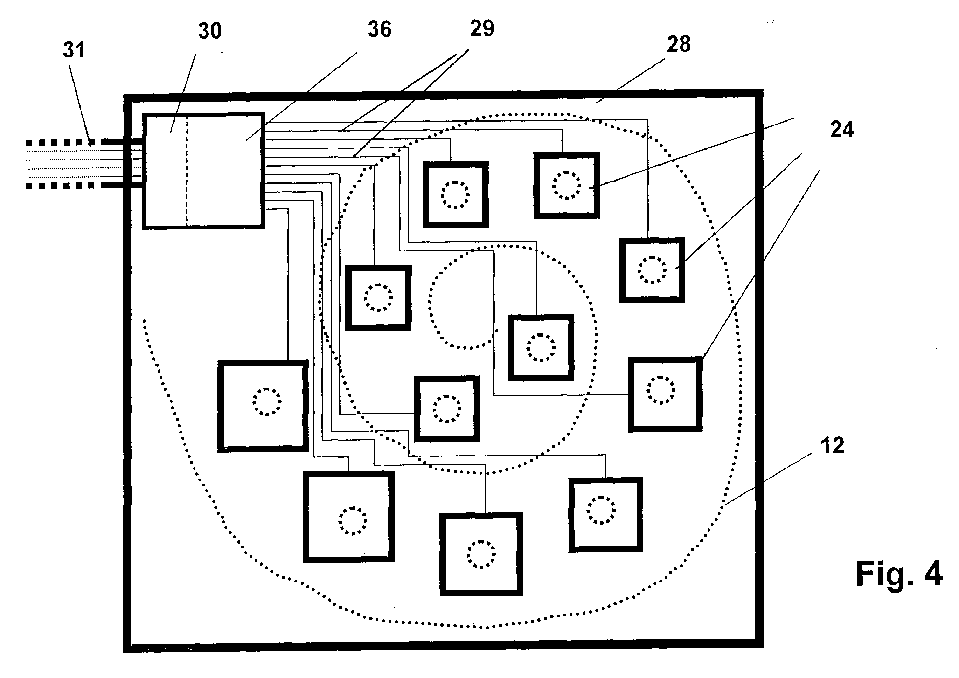

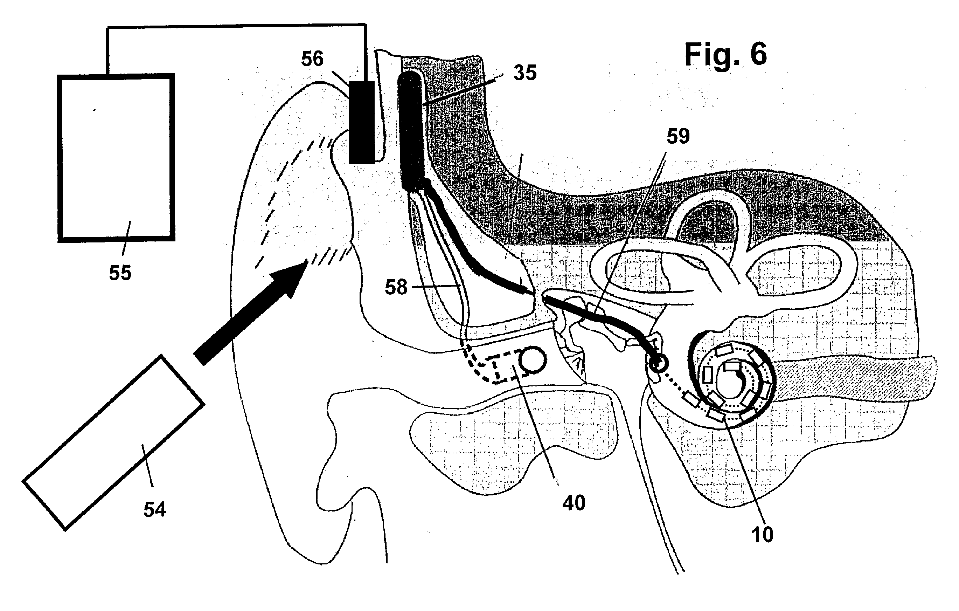

Fig. 4 zeigt schematisch einen möglichen Aufbau des extracochleären elektromechanischen Wandler-Arrays 22 gemäß Fig. 3, der bevorzugt mit mikrosystemtechnischen Methoden hergestellt werden kann. Auf einer Trägerplatte (Substrat) 28 sind mehrere Wandlereinheiten 24 (zum Beispiel nach WO-A-99/3146) geometrisch so verteilt angeordnet, wie es dem statistischen Mittelwert der geometrischen Cochlea-Dimensionen entspricht (gepunktet unterlegter Umriß der Cochlea 12). Dieses Substrat 28 beinhaltet gleichzeitig die elektrischen Wandlerzuleitungen 29 (in Fig. 4 angedeutet). Weiterhin ist ein elektrisches Anschlußfeld 30 vorgesehen, das mikrosystemtechnisch mitgefertigt wird und den adäquaten Anschluß einer mehrpoligen, biokompatiblen Implantatleitung 31 zu einem in den Figuren 6 bis 10 veranschaulichten Elektronikmodul 34 ermöglicht, das die treibende Signalverarbeitungselektronik des implantierbaren Hörsystems enthält. Darüberhinaus kann das Substrat 28 ein mikrosystemtechnisch mitgefertigtes Elektronikmodul 36 enthalten, das beispielhaft die Wandler 24 ansteuernde Treiberstufen enthalten kann. Vorteilhaft kann dieses Modul 36 auch eine Dekodierlogik und Umsetzerbausteine enthalten, die den Anschluß einer polreduzierten Implantatleitung ermöglichen. So kann beispielhaft der Arrayanschluß aus nur drei Leitungen bestehen (Masse, Daten und ein Taktsignal), wobei die notwendige Versorgung mit elektrischer Betriebsenergie durch Phantomspeisung auf der Taktsignalleitung erfolgen kann. Die wandlertreibenden Signale liegen dann in digital kodierter Form vor, die mit hoher Taktrate seriell übertragen werden. Weiterhin kann ein Schnittstellenbaustein enthalten sein, der die digitale Datenübertragung über die Implantatleitung vorteilhaft mittels einer Lichtleitfaser ermöglicht. Bei serieller Datenübertragung sind in dem Elektronikmodul 36 entsprechende den Wandlern 24 zugeordnete D/A-Wandler und Treiberbausteine enthalten. Das gesamte Wandler-Array 22 einschließlich Trägeraufbau (Substrat) 28 ist mit einer biokompatiblen Ummantelung ausgestattet, die zum Beispiel aus bekannten Polymeren aus der Implantattechnologie besteht (Polytetrafluorethylen, Polyurethan, Silikone).

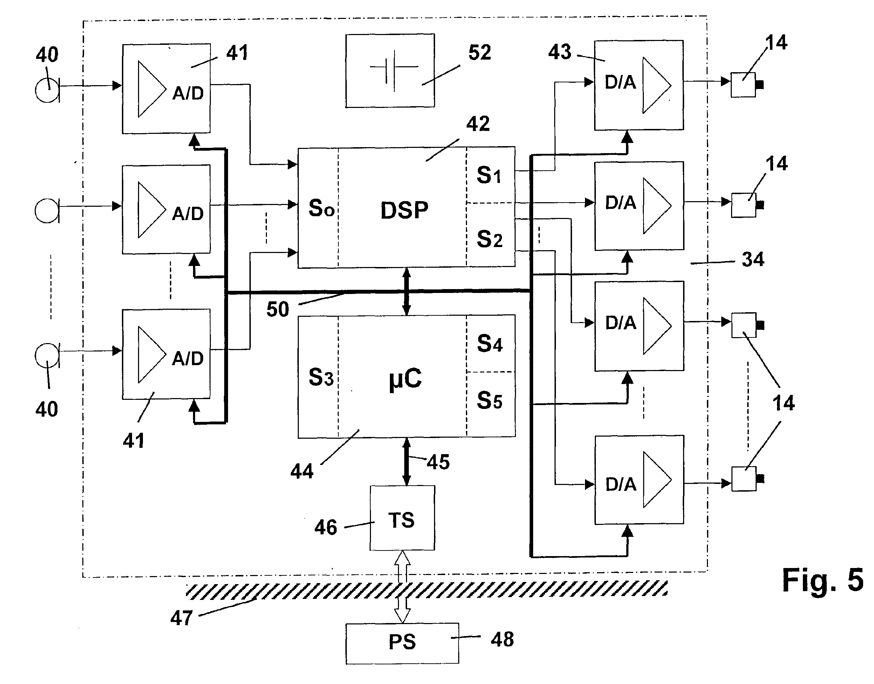

Fig. 5 zeigt den möglichen Aufbau des signalverarbeitenden Elektronikmoduls 34 des mindestens teilweise implantierbaren Hörsystems. Über einen oder mehrere Schallsensoren (Mikrofone) 40 wird das externe Schallsignal aufgenommen und in elektrische Signale umgewandelt. Die analogen elektrischen Sensorsignale werden an Module 41 geleitet, wo sie vorverarbeitet, insbesondere vorverstärkt, und in digitale Signale umgewandelt (A/D) werden. Diese Vorverarbeitung kann beispielsweise in einer analogen linearen oder nichtlinearen Vorverstärkung und Filterung (zum Beispiel Antialiasing-Filterung) bestehen.

Die digitalisierten Sensorsignale werden in einem digitalen Signalprozessor 42 (DSP) weiterverarbeitet. Der Signalprozessor 42 enthält einen nicht überschreibbaren Festspeicherbereich S₀, in welchem die für einen "Minimalbetrieb" des Systems erforderlichen Anweisungen und Parameter gespeichert sind, sowie Speicherbereiche S₁ und S₂, in denen die Betriebssoftware der bestimmungsgemäßen Funktion beziehungsweise Funktionen des Implantatsystems abgelegt sind. Die wiederholt beschreibbaren Programmspeicher S₁ und S₂ zur Aufnahme der Betriebssoftware können auf EEPROM-Basis oder statischen RAM-Zellen basieren, wobei im letztgenannten Fall dafür gesorgt werden sollte, daß dieser RAM-Bereich immer durch das implantatinterne Energieversorgungssystem "gepuffert" ist.

Die digitalen Ausgangssignale des Signalprozessors 42 werden in Digital-Analog-Wandlern (D/A) 43 in Analogsignale umgewandelt und verstärkt sowie dann den ausgangsseitigen elektromechanischen Wandlern 14 zugeführt. Die D/A-Wandler 43 können gegebenenfalls entfallen, wenn beispielsweise bei einem Hörsystem mit elektromagnetischem Ausgangswandler ein zum Beispiel pulsweitenmoduliertes, serielles digitales Ausgangssignal des Signalprozessors 42 direkt an die Ausgangswandler 14 übermittelt wird.

Der Signalprozessor 42 führt die bestimmungsgemäße Funktion des Hörimplantates aus. Dazu gehören eine Audiosignalverarbeitung für die Rehabilitation einer Hörstörung und gegebenenfalls auch eine Signalgenerierung im Fall eines Systems mit zusätzlicher Tinnitusmaskierer- oder Noiser-Funktion. Weiterhin enthält der digitale Signalprozessor 42 Softwaremodule, die eine solche Ansteuerung der ausgangsseitigen elektromechanischen Wandler 14 bewirken, daß die spektralen, zeitlichen, amplituden- und phasenbezogenen Wandlersignaleigenschaften so bemessen sind, daß auf der Basilarmembran des geschädigten Innenohres eine Wanderwelle erzeugt wird, die der des gesunden Gehörs möglichst nahe kommt. Diese Softwaremodule können statisch und dynamisch ausgelegt sein. Unter statischer Auslegung soll dabei verstanden werden, daß die Softwaremodule aufgrund wissenschaftlicher Erkenntnisse einmalig im Programmspeicher des Signalprozessors 42 abgelegt werden und unverändert bleiben. Dynamisch bedeutet, daß diese Softwaremodule "lernfähig" sind, um der angestrebten Wanderwellenkonfiguration zeitlich iterativ möglichst optimal nahe zu kommen. Dies bedeutet, daß die Softwaremodule adaptiv ausgelegt sein können und die Parameteranpassung durch "Training" durch den Implantatträger und gegebenenfalls weitere Hilfsmittel, wie Rehabilitationsprogramme, vorgenommen wird. Weiterhin kann ein Softwaremodul enthalten sein, welches eine möglichst optimale Simulation des "gesunden" cochleären Verstärkers auf der Basis eines lernfähigen neuronalen Netzwerkes approximiert. Das Training dieses neuronalen Netzes kann wieder durch den Implantatträger und/oder unter Zuhilfenahme weiterer externer Hilfsmittel erfolgen.

Eine Methode zur möglichst optimalen Simulation des "gesunden" cochleären Verstärkers kann die Implementierung des Prinzips der "Time-Reversed Acoustics" (TRA) sein (Fink, M.: "Time-Reversed Acoustics", Scientific American 281:5 (1999), p. 67-73). Die Ansteuerung der ausgangsseitigen Wandlerelemente 14 erfolgt mittels TRA so, daß örtlich begrenzte Bereiche der Cochlea mechanisch stimuliert werden. Während in herkömmlichen Anwendungen von TRA die Registrierung des verteilten Schallereignisses und die Aussendung des time-reversed Signals im selben Präparat erfolgen, werden diese beiden Schritte im vorliegenden Fall getrennt. Die verteilten Ereignisse können zum Beispiel in einem adäquaten Tiermodell intracochleär ermittelt werden; anschließend werden die time-reversed Stimuli bei der vorliegenden Applikation eines Hörsystems für den Menschen, gegebenenfalls unter Parameteranpassung an die veränderte Geometrie der menschlichen Cochlea, appliziert.

Damit die beschriebenen, softwarebasierten Algorithmen zur möglichst optimalen Simulation des cochleären Verstärkers insbesondere in einem Vollimplantat auch postoperativ implementiert werden können, enthält das System nach Fig. 5 einen weiteren Mikroprozessorbaustein, zum Beispiel einen Mikrokontroller (µC) 44 mit zugehörigen Speichern S₃, S₄ und S₅. Bei dem Speicher S₃ handelt es sich um einen wiederholt beschreibbaren Speicher, in welchem ein Arbeitsprogramm für den Microcontroller 44 abgelegt ist. In den Speicherbereichen S₄ und S₅ können insbesondere die Betriebsoftwareanteile des Implantatmanagementsystems abgelegt sein (zum Beispiel Verwaltungsüberwachungsund Telemetriefunktionen). In den Speichern S₁ und/oder S₂ und/oder S₄ und/oder S₅ können auch von außen veränderliche, patientenspezifische wie zum Beispiel audiologische Anpaßparameter, abgelegt sein.

Der Mikrokontroller 44 kommuniziert einerseits über einen bidirektionalen Datenbus 45 und ein Telemetriesystem (TS) 46 drahtlos (beispielsweise mittels induktiver Kopplung) durch die bei 47 angedeutete geschlossene Haut mit einem externen Programmiersystem (PS) 48. Das Programmiersystem 48 kann ein PC-basiertes System mit entsprechender Programmier-, Bearbeitungs-, Darstellungs- und Verwaltungssoftware sein. Über diese Telemetrieschnittstelle wird die zu verändernde beziehungsweise ganz auszutauschende Betriebssoftware des Implantatsystems übertragen und zunächst in dem Speicherbereich S₄ und/oder S₅ des Mikrokontrollers 44 zwischengespeichert. So kann zum Beispiel eine einfache Verifikation der Softwareübertragung durch einen Lesevorgang über die Telemetrieschnittstelle durchgeführt werden, bevor die Betriebssoftware oder die entsprechenden Signalverarbeitungsanteile dieser Software in die Programmspeicherbereiche S₁, und S₂ des digitalen Signalprozessors 42 über einen Datenbus 50 übertragen werden.

Ferner kann auch das Arbeitsprogramm für den Microcontroller 44 über die Telemetrieschnittstelle ganz oder teilweise mit Hilfe der externen Einheit 48 geändert oder ausgetauscht werden.

Andererseits steuert der Mikrokontroller 44 implantatintern über den bidirektionalen Datenbus 50 die A/D-Wandler 41 der Sensor-Vorverarbeitung, die D/A-Wandler 43 zur Ansteuerung der ausgangsseitigen elektromechanischen Wandler 14 und den Signalprozessor 42 selbst. Über den Datenbus 50 werden auch Programmteile oder ganze Softwaremodule zwischen Außenwelt, Mikrokontroller 44 und Signalprozessor 42 übermittelt.

Das Implantatsystem enthält bei vollimplantierter Ausführungsform auch eine primäre oder sekundäre Batteriezelle 52, die die einzelnen Module mit elektrischer Betriebsenergie versorgt.

Fig. 6 zeigt schematisch den Aufbau eines vollständig implantierbaren Hörsystems mit einem intracochleären Wandler-Array 10 gemäß Fig. 1 und einem implantierbaren Mikrofon 40. Eine drahtlose Fernbedienung 54 dient der Steuerung der Implantatfunktionen durch den Implantatträger. Ferner ist ein Ladesystem mit einem Ladegerät 55 zum drahtlosen transkutanen Nachladen einer im Implantat befindlichen sekundären Batterie zur Energieversorgung des Hörsystems, beispielsweise der Batterie 52 in Fig. 5, vorgesehen.

Das Mikrofon 40 kann vorteilhaft in der aus US-A-5 814 095 bekannten Weise aufgebaut und mit einer Mikrofonkapsel, die in einem Gehäuse allseitig hermetisch dicht untergebracht ist, sowie mit einer elektrischen Durchführungsanordnung zum Durchführen mindestens eines elektrischen Anschlusses von dem Innenraum des Gehäuses zu dessen Außenseite versehen sein, wobei das Gehäuse mindestens zwei Schenkel aufweist, die in einem Winkel mit Bezug aufeinander ausgerichtet sind, wobei der eine Schenkel die Mikrofonkapsel aufnimmt und mit einer Schalleintrittsmembran versehen ist, wobei der andere Schenkel die elektrische Durchführungsanordnung enthält und gegenüber der Ebene der Schalleintrittsmembran zurückversetzt ist, und wobei die Geometrie des Mikrofongehäuses so gewählt ist, daß bei Implantation des Mikrofons in der Mastoidhöhle der die Schalleintrittsmembran enthaltende Schenkel vom Mastoid aus in eine artifizielle Bohrung in der hinteren, knöchernen Gehörgangswand hineinragt und die Schalleintrittsmembran die Haut der Gehörgangswand berührt. Zur Festlegung des Mikrofons 40 kann zweckmäßig ein Fixationselement der aus US-A-5 999 632 bekannten Art vorgesehen sein, das eine Manschette aufweist, die mit einem zylindrischen Gehäuseteil den die Schalleintrittsmembran enthaltenden Schenkel umschließt und mit gegen die der Gehörgangshaut zugewendete Seite der Gehörgangswand anlegbaren, vorspringenden, elastischen Flanschteile versehen ist. Dabei beinhaltet das Fixationselement vorzugsweise eine Halterung, welche die genannten Flanschteile vor der Implantation entgegen einer elastischen Rückstellkraft der Flanschteile in einer das Durchstecken durch die Bohrung der Gehörgangswand erlaubenden umgebogenen Stellung hält.

Zu dem Ladesystem gehört auch eine an den Ausgang des Ladegerätes 55 angeschlossene Ladespule 56, die vorzugsweise in der aus US-A-5 279 292 bekannten Art Teil eines Sende-Serienresonanzkreises bildet, der mit einem nicht veranschaulichten Empfangs-Serienresonanzkreis induktiv gekoppelt werden kann. Der Empfangs-Serienresonanzkreis kann bei der Ausführungsform gemäß Fig. 6 Teil des Elektronikmoduls 34 sein und entsprechend US-A-5 279 292 eine Konstantstromquelle für die Batterie 52 (Fig. 5) bilden. Dabei liegt der Empfangs-Serienresonanzkreis in einem Batterie-Ladestromkreis, der in Abhängigkeit von der jeweiligen Phase des in dem Ladestromkreis fließenden Ladestromes über den einen oder den anderen Zweig einer Vollweg-Gleichrichterbrücke geschlossen wird.

Das Elektronikmodul 34 ist bei der Anordnung nach Fig. 6 über eine Mikrofonleitung 58 an das Mikrofon 40 und über eine Wandler-Array-Zuleitung 59 an das intracochleäre Wandler-Array 10 angeschlossen.

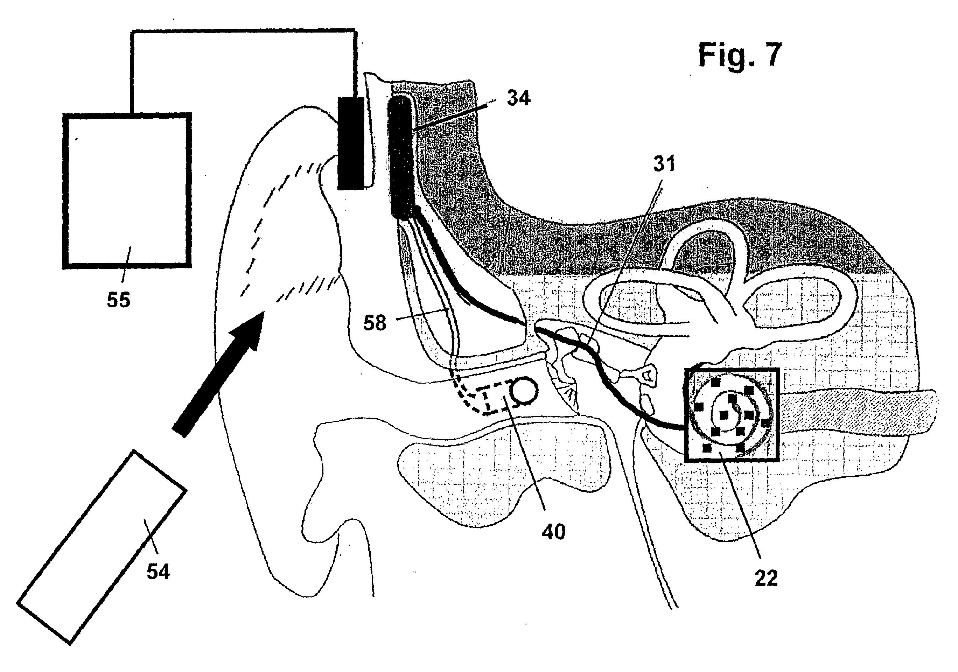

Fig. 7 zeigt schematisch den Aufbau eines vollständig implantierbaren Hörsystems mit einem extracochleären Wandler-Array 22 gemäß den Figuren 3 und 4 und dem implantierbaren Mikrofon 40. Die drahtlose Fernbedienung 54 ist auch in diesem Fall zur Steuerung der Implantatfunktionen durch den Implantatträger vorgesehen, und ein Ladesystem mit Ladegerät 55 und Ladespule 56 entsprechend Fig. 6 dient dem drahtlosen, transkutanen Nachladen einer im Implantat befindlichen sekundären Batterie 52 zur Energieversorgung des Hörsystems. Das Wandler-Array 22 steht mit dem Elektronikmodul 34 über die Implantatleitung 31 in Verbindung.

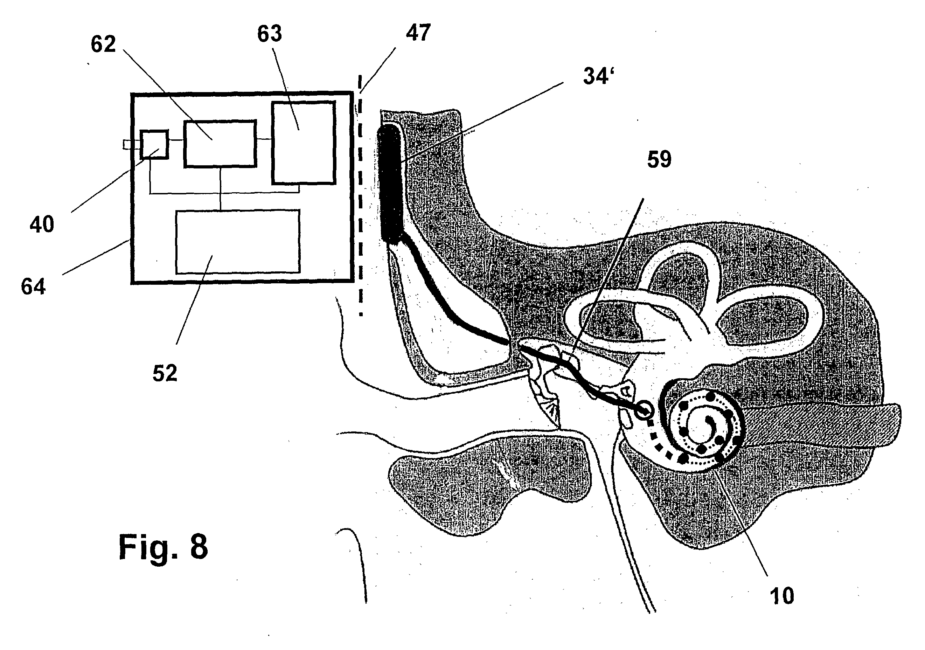

Fig. 8 zeigt schematisch den Aufbau eines teilimplantierbaren Hörsystems mit einem intracochleären Wandler-Array 10 gemäß Fig. 1. Bei diesem teilimplantierbaren System sind ein Mikrofon 40, ein Elektronikmodul 62 für eine elektronische Signalverarbeitung weitestgehend entsprechend Fig. 5 (aber ohne das Telemetriesystem 46), die Energieversorgung 52 sowie eine Modulator/Sender-Einheit 63 in einem extern am Körper, vorzugsweise am Kopf über dem Implantat, zu tragenden externen Modul 64 enthalten. Das Implantat ist wie bei bekannten Teilimplantaten energetisch passiv. Sein Elektronikmodul 34' (ohne Batterie 52) empfängt seine Betriebsenergie und Wandlersteuerdaten über die Modulator/Sender-Einheit 63 im externen Teil 64. Es versteht sich, daß bei einem solchen teilimplantierbaren Hörsystem das Wandler-Array auch extracochleär gemäß den Figuren 3 und 4 ausgeführt sein kann.

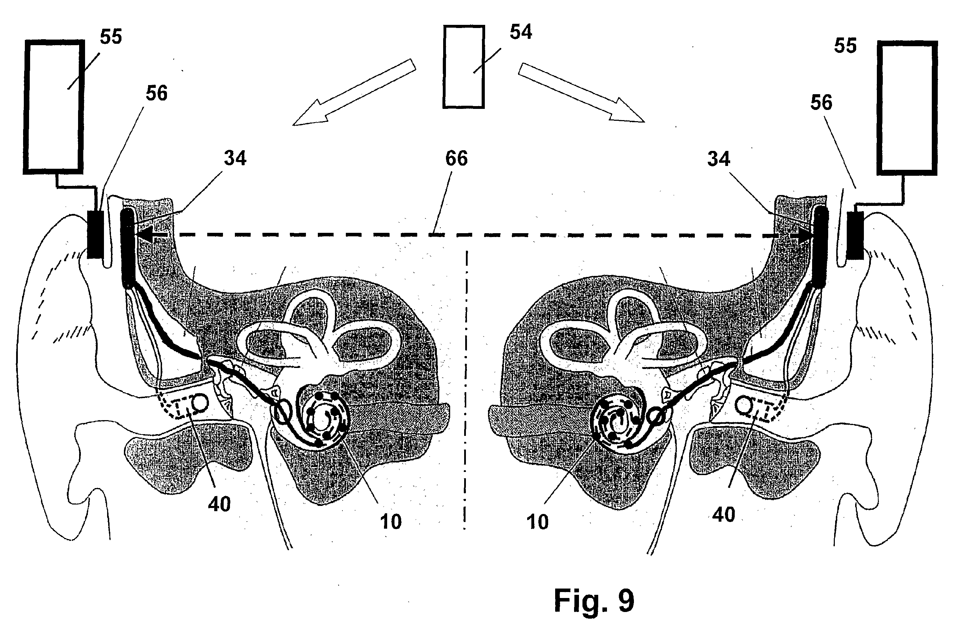

Fig. 9 zeigt eine binaurale Anwendung eines Hörimplantates (hier mit intracochleären Wandler-Arrays 10), bei dem die Signalverarbeitungsmodule 34 über eine drahtgebundene implantierbare Leitungsverbindung 66 so miteinander kommunizieren, daß eine optimale binaurale Signalverarbeitung und Wandler-Array-Ansteuerung in beiden implantierten Innenohren erreicht wird. Weiterhin sind auch hier transkutane Ladevorrichtungen 55, 56 für den Fall implantatseitiger sekundärer Energiespeicherelemente (Batterien 52) sowie eine beide Elektronikmodule 34 synchron steuernde drahtlose Fernbedienung 54 zur Anwendung durch den Implantatträger vorgesehen.

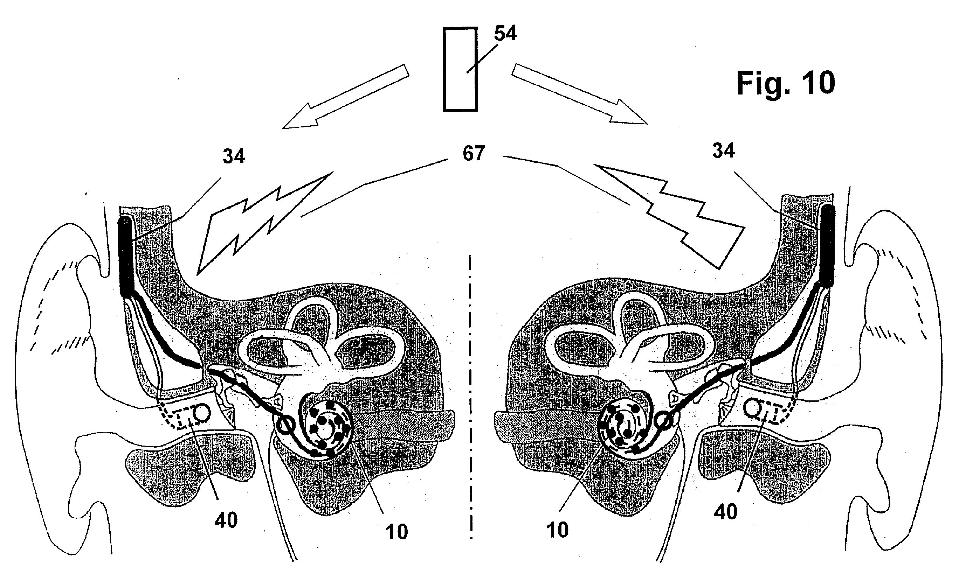

Fig. 10 zeigt die binaurale Anwendung eines Hörimplantates (hier mit intracochleären Wandler-Arrays 22), bei dem die Signalverarbeitungsmodule 34 über eine drahtlose Verbindung (zum Beispiel eine bei 67 angedeutete bidirektionale Hochfrequenzstrecke) so miteinander kommunizieren, daß eine optimale binaurale Signalverarbeitung und Wandler-Array-Ansteuerung in beiden implantierten Innenohren erreicht wird. Weiterhin sind auch hier transkutane Ladevorrichtungen 55, 56 (nicht dargestellt) für den Fall implantatseitiger sekundärer Energiespeicherelemente (Batterien 52) vorgesehen sowie eine drahtlose Fernbedienung 54 zur Anwendung durch den Implantatträger, die beide Elektronikmodule 34 synchron steuert.

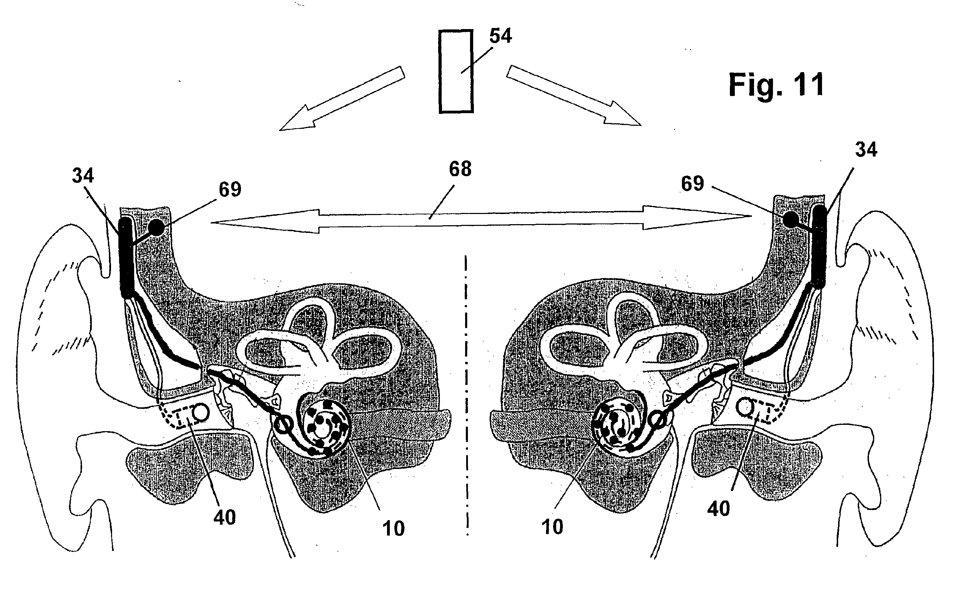

Die binaurale Ausbildung des Hörimplantates gemäß Fig. 11 unterscheidet sich von denjenigen der Fig. 10 nur dadurch, daß für die drahtlose Kommunikation zwischen den Signalverarbeitungsmodulen 34 beider Systemeinheiten eine körperschallgekoppelte Ultraschallstrecke 68 mit Ultraschall-Kopplern 69 vorgesehen ist. Dabei sind die zum Beispiel digitalen, bidirektionalen Informationen auf einen Träger im Ultraschallbereich vorzugsweise amplituden- oder frequenzmoduliert. Die Ultraschall-Koppler 69 können, wie in Fig. 11 dargestellt, von dem Elektronikmodul 34 örtlich getrennte und über elektrische Leitungen angeschlossene Ultraschallempfänger und -sender sein, die vorzugsweise im Mastoidbereich an den Schädelknochen fest angekoppelt sind. Die Ultraschall-Koppler können aber auch in den Elektronikmodulen 34 integriert sein (nicht dargestellt), wenn die Elektronikmodule so im Mastoidbereich implantiert werden, daß eine Ultraschall-Körperübertragung durch den Schädelknochen stattfinden kann.

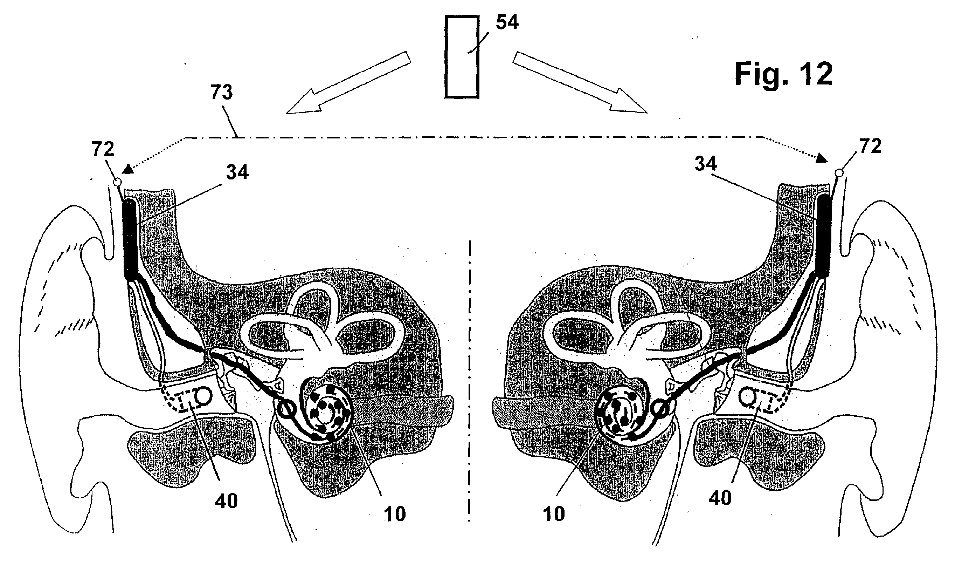

Eine weiter abgewandelte Ausführungsform eines binaural ausgebildeten Hörimplantates ist in Fig. 12 dargestellt. Bei dieser Ausführungsform werden abweichend von den Ausführungsbeispielen der Figuren 9 bis 11 die zum Beispiel digitalen, bidirektionalen Informationen implantatseitig auf einen Träger vorzugsweise amplituden- oder frequenzmoduliert und an implantierte Elektroden 72 angelegt, die Teil einer durch Körpergewebe des Implantatträgers führenden Datenübertragungsstrecke 73 sind. Es wird so ein modulierter Gewebestrom erhalten, der in an sich bekannter Weise (DE-A-38 31 809) für die gewünschte Kommunikation zwischen den Signalverarbeitungsmodulen 34 beider Systemeinheiten sorgt.

Es versteht sich, daß auch ein teilimplantierbares System binaural appliziert werden kann und daß dann für eine Kommunikation zwischen den beiden Systemeinheiten vorzugsweise entsprechend den in den Figuren 9 bis 12 veranschaulichen Ausführungsbeispielen von binauralen Anwendungen vollimplantierbarer Systeme gesorgt werden kann.

Fig. 13 zeigt ein weiteres Ausführungsbeispiel für einen vorliegend geeigneten intracochleären elektromechanischen Wandler. Die Wandlerausbildung gemäß Fig. 13 entspricht derjenigen der Fig. 2 mit der Ausnahme, daß der in den Träger 11 eingebettete Wandler 14' als Hohlzylinder ausgebildet ist. Das Anlegen eines Wandlerwechselspannungssignals bewirkt eine mit unterbrochenen Linien angedeutete dynamische Volumenänderung des Wandlers. Dabei wird der Umstand ausgenutzt, daß eine Volumenverkleinerung des hohlzylindrischen Wandlers 14' das Innenvolumen des Wandlers verringert und infolgedessen dort befindliche intracochleäre Flüssigkeit 76 aus dem Zylinderhohlraum 77 herausgepreßt wird, wie dies mit den Pfeilen 79 angedeutet ist. In dem Träger 11 sind benachbart den axialen Wandlerenden Austrittsöffnungen 82 vorgesehen, aus denen intracochleäre Flüssigkeit (Pfeile 79) herausspritzt, wenn der Wandler 14' aufgrund eines angelegten Spannungssignals eine Volumenverkleinerung erfährt und dementsprechend auf die intracochleäre Flüssigkeit die vorstehend geschilderte Pumpwirkung ausgeübt wird. Durch entsprechende Bemessung des Verhältnisses zwischen der sich vergrößernden und verkleinernden Wandleroberfläche und der Öffnungsfläche der Austrittsöffnungen 82 kann eine Schnelletransformation erreicht werden, die schon bei kleinen Volumenänderungen der Wandler 14' relativ hohe Druckänderungen im Bereich der Austrittsöffnungen 82 entstehen läßt.

## Patentansprüche

1. Mindestens teilweise implantierbares System zur Rehabilitation einer Hörstörung, das mindestens einen Sensor (Mikrofon) (40) zur Aufnahme des Schallsignals und dessen Umwandlung in entsprechende elektrische Signale, eine elektronische Signalverarbeitungseinheit (34, 34', 62) zur Audiosignalverarbeitung und -verstärkung, eine elektrische Energieversorgungseinheit (52), welche einzelne Komponenten des Systems mit Strom versorgt, sowie eine ausgangsseitige elektromechanische Wandleranordnung (10, 22) umfasst, die aus mindestens zwei voneinander unabhängigen und örtlich getrennt angeordneten Wandlern (14, 24) zum Stimulieren des Innenohres besteht, **dadurch gekennzeichnet, daß** die ausgangsseitigen elektromechanischen Wandler (14, 14', 24) zur Anregung der flüssigkeitsgefüllten Innenohrräume des geschädigten Innenohrs ausgelegt sind, und daß die Signalverarbeitungseinheit (34, 34', 62) eine treibende Signalverarbeitungselektronik (42, 44) aufweist, die jeden der Wandler derart elektrisch ansteuert, daß auf der Basilarmembran des geschädigten Innenohres eine Wanderwellenkonfiguration entsteht, die die Art der Wanderwellenausbildung eines gesunden, nicht geschädigten Innenohres approximiert.

2. System nach Anspruch 1, **dadurch gekennzeichnet, daß** die ausgangsseitigen elektromechanischen Wandler (14, 14', 24) für eine direkte Anregung der flüssigkeitsgefüllten Innenohrräume des geschädigten Innenohrs ausgelegt sind.

3. System nach Anspruch 2, **gekennzeichnet durch** ein intracochleäres Array (10) von ausgangsseitigen elektromechanischen Wandlern (14, 14').

4. System nach Anspruch 3, **dadurch gekennzeichnet, daß** der Gesamtdurchmesser des intracochleären Wandler-Arrays (10) im Bereich von 0,4 mm (apikaler Bereich) bis 2,0 mm (basaler Bereich) liegt.

5. System nach Anspruch 3 oder 4, **dadurch gekennzeichnet, daß** die Gesamtlänge des intracochleären Wandler-Arrays (10) zwischen 5 mm und 50 mm liegt.

6. System nach einem der Ansprüche 3 bis 5, **dadurch gekennzeichnet, daß** das intracochleäre Wandler-Array (10) einen Träger (11) aus einem biokompatiblen und im Innenohr biostabilen Material, vorzugsweise einem Polymer, insbesondere einem Silikon, aufweist.

7. System nach Anspruch 6, **dadurch gekennzeichnet, daß** die einzelnen ausgangsseitigen elektromechanischen Wandler (14, 14') so in den Träger (11) eingebettet sind, daß sie von einer dünnen Schicht des Trägermaterials vollständig umgeben sind.

8. System nach Anspruch 6 oder 7, **dadurch gekennzeichnet, daß** zwischen den einzelnen ausgangsseitigen elektromechanischen Wandlern (14, 14') mechanische Dämpfungselemente (20) in den Träger (11) eingebettet sind, die die mechanische Wellenausbreitung innerhalb des Trägers zu benachbarten Wandlern minimieren.

9. System nach Anspruch 8, **dadurch gekennzeichnet, daß** das Material der Dämpfungselemente (20) bei ähnlicher Querschnittsgeometrie wie die des Trägers (11) so gewählt ist, daß zur Erzielung hoher Dämpfungswerte ein großer mechanischer Impedanzunterschied zu dem Trägermaterial besteht.

10. System nach Anspruch 1 oder 2, **gekennzeichnet durch** ein extracochleäres Array (22) von ausgangsseitigen elektromechanischen Wandlern (24).

11. System nach Anspruch 10, **dadurch gekennzeichnet, daß** das extracochleäre Wandler-Array (22) mikrosystemtechnisch hergestellt ist.

12. System nach Anspruch 11, **dadurch gekennzeichnet, daß** für das extracochleäre Wandler-Array (22) ein Substrat (28) vorgesehen ist, das ein elektrisches Anschlußfeld (30) enthält, das mikrosystemtechnisch mitgefertigt ist und für den Anschluß einer mehrpoligen, biokompatiblen Implantatleitung (31) zu einem Modul (34, 34') ausgelegt ist, das die treibende Signalverarbeitungselektronik enthält.

13. System nach Anspruch 11 oder 12, **dadurch gekennzeichnet, daß** für das extracochleäre Wandler-Array (22) ein Substrat (28) mit einem mikrosystemtechnisch mitgefertigten Elektronikmodul (36) vorgesehen ist.

14. System nach Anspruch 13, **dadurch gekennzeichnet, daß** das Elektronikmodul (36) Treiberstufen zum Ansteuern der ausgangsseitigen elektromechanischen Wandler (24) enthält.

15. System nach Anspruch 13 oder 14, **dadurch gekennzeichnet, daß** das Elektronikmodul (36) eine Dekodierlogik und Umsetzerbausteine für den Anschluß einer polreduzierten Implantatleitung (31) enthält.

16. System nach Anspruch 15, **dadurch gekennzeichnet, daß** der Array-Anschluß aus nur drei Leitungen, insbesondere einer Masseleitung, einer Datenleitung und einer Taktsignalleitung, besteht und die Versorgung mit elektrischer Betriebsenergie durch Phantomspeisung auf der Taktsignalleitung erfolgt.

17. System nach einem der Ansprüche 13 bis 16, **dadurch gekennzeichnet, daß** das Elektronikmodul (36) einen Schnittstellenbaustein für eine digitale Datenübertragung über die Implantatleitung (31), insbesondere mittels einer Lichtleitfaser, enthält.

18. System nach einem der Ansprüche 13 bis 17, **dadurch gekennzeichnet, daß** das Elektronikmodul (36) bei serieller Datenübertragung auf der Implantatzuleitung (31) entsprechende den Wandlern (24) zugeordnete D/A-Wandler und Treiberbausteine enthält.

19. System nach einem der Ansprüche 13 bis 18, **dadurch gekennzeichnet, daß** das extracochleäre Wandler-Array (22) einschließlich Trägeraufbau (Substrat) (28) mit einer biokompatiblen Ummantelung ausgestattet ist, die vorzugsweise aus von der Implantattechnologie bekannten Polymeren, insbesondere Polytetrafluorethylen, Polyurethan, oder Silikonen, besteht.

20. System nach Anspruch 2 und einem der Ansprüche 10 bis 19, **dadurch gekennzeichnet, daß** die Wandler (24) des extracochleären Wandler-Arrays (22) jeweils ein ausgangsseitiges Koppelelement (26) aufweisen, das so ausgebildet ist, daß es durch eine Öffnung der Cochlea-Wand in einen flüssigkeitsgefüllten Innenohrraum ragen kann.

21. System nach einem der vorhergehenden Ansprüche, **dadurch gekennzeichnet, daß** die ausgangsseitigen elektromechanischen Wandler (14, 14', 24) als elektromagnetische, elektrodynamische, piezoelektrische, magnetostriktive oder dielektrische (kapazitive) Wandler ausgebildet sind.

22. System nach Anspruch 13, **dadurch gekennzeichnet, daß** die ausgangsseitigen elektromechanischen Wandler (14, 14', 24) bei Anwendung des piezoelektrischen Wandlerprinzips unter Verwendung von PZT-Keramik (Blei-Zirkonat-Titanat) oder PVDF (Polyvinylidenfluorid) aufgebaut sind.

23. System nach einem der vorhergehenden Ansprüche, **dadurch gekennzeichnet, daß** die ausgangsseitigen elektromechanischen Wandler (14, 14', 24), vorzugsweise mit Ausnützung von geometrischen Gestalttransformationen, insbesondere des Bimorph-Prinzips, des Unimorph-Prinzips oder des Heteromorph-Prinzips mit passiven Materialpartnern, so ausgeführt sind, daß sie bei gegebener Wandlerspannung eine maximale Auslenkung bei minimaler elektrischer Leistungsaufnahme erzeugen.

24. System nach einem der Ansprüche 3 bis 23, **dadurch gekennzeichnet, daß** die ausgangsseitigen elektromechanischen Wandler (14, 14', 24) in dem Wandler-Array (10, 22) äquidistant oder in logarithmischen Distanzen - entsprechend der tonotopen Frequenz-Orts-Zuordnung längs der Basilarmembran des Innenohres - verteilt angeordnet sind.

25. System nach Anspruch 24, **dadurch gekennzeichnet, daß** bei tonotoper Anordnung der ausgangsseitigen elektromechanischen Wandler (14, 14', 24) eine Wandleranzahl von 20 - 24 gemäß den psychoakustischen Frequenzgruppen vorgesehen ist.

26. System nach einem der vorhergehenden Ansprüche, **dadurch gekennzeichnet, daß** die ausgangsseitigen elektromechanischen Wandler (14, 14', 24) einen Übertragungsbereich von etwa 100 Hz bis etwa 10 kHz haben.

27. System nach einem der vorhergehenden Ansprüche, **dadurch gekennzeichnet, daß** die ausgangsseitigen elektromechanischen Wandler (14, 14', 24) so abgestimmt sind, daß ihre erste mechanische Resonsanzfrequenz am oberen Ende des angestrebten Übertragungsfrequenzbereiches, insbesondere bei etwa 8 kHz bis etwa 10 kHz, liegt.

28. System nach einem der vorhergehenden Ansprüche, **dadurch gekennzeichnet, daß** die ausgangsseitigen elektromechanischen Wandler (14, 14', 24) hermetisch dicht aufgebaut sind.

29. System nach einem der vorhergehenden Ansprüche, **dadurch gekennzeichnet, daß** die Signalverarbeitungseinheit (34, 34', 62) eine Vorverarbeitungsanordnung (41) zum Vorverstärken und/oder Filtern sowie zum Analog-Digital- (A/D-) Wandeln der Schallsensorsignale aufweist.

30. System nach Anspruch 29, **dadurch gekennzeichnet, daß** die Vorverarbeitungsanordnung einen Antialiasing-Filter umfaßt.

31. System nach einem der vorhergehenden Ansprüche, **dadurch gekennzeichnet, daß** bei Vorhandensein mehrerer Schallsensoren (40) jedem der Schallsensoren ein Analog-Digital-Wandler (41) nachgeschaltet ist.

32. System nach einem der vorhergehenden Ansprüche, **dadurch gekennzeichnet, daß** die Signalverarbeitungseinheit (34, 34', 62) Softwaremodule enthält, die parallel zum Hörgerätebetrieb die Maskierung eines Tinnitus ermöglichen.

33. System nach einem der vorhergehenden Ansprüche, **dadurch gekennzeichnet, daß** die Signalverarbeitungseinheit (34, 34', 62) einen digitalen Signalprozessor (42) zum Verarbeiten der A/D-gewandelten und gegebenenfalls mittels der Vorverarbeitungsanordnung (41) vorverarbeiteten Schallsensorsignale und/oder zum Generieren von digitalen Signalen für eine Tinnitusmaskierung aufweist.

34. System nach einem der vorhergehenden Ansprüche, **gekennzeichnet durch** mindestens einen der ausgangsseitigen elektromechanischen Wandleranordnung (10, 22) vorgeschalteten Digital-Analog-Wandler (43).

35. System nach Anspruch 34, **dadurch gekennzeichnet, daß** den ausgangsseitigen elektromechanischen Wandlern (14, 14', 24) jeweils ein eigener Digital-Analog-Wandler (43) vorgeschaltet ist.

36. System nach einem der vorhergehenden Ansprüche, **dadurch gekennzeichnet, daß** die Signalverarbeitungselektronik (42, 44) Softwaremodule enthält, die die Ansteuerung der ausgangsseitigen elektromechanischen Wandler (14, 14', 24) so vornehmen, daß die spektralen, zeitlichen, amplituden- und phasenbezogenen Wandlersignaleigenschaften so bemessen sind, daß eine Wanderwelle auf der Basilarmembran des geschädigten Innenohres erzeugt wird, die der eines gesunden Gehörs möglichst nahe kommt.

37. System nach Anspruch 36, **dadurch gekennzeichnet, daß** die Softwaremodule statisch in der Weise ausgelegt sind, daß sie aufgrund wissenschaftlicher Erkenntnisse einmalig in einem Programmspeicher (S₁, S₂) des digitalen Signalprozessors (42) abgelegt werden und unverändert bleiben.

38. System nach einem der vorhergehenden Ansprüche, **gekennzeichnet durch** eine vorzugsweise PC-basierte, drahtlose Telemetrieeinrichtung (46) zur Übertragung von Daten zwischen dem implantierten Teil (34) des Systems und einer externen Einheit, insbesondere einem externen Programmiersystem (48).

39. System nach Ansprüchen 36 und 38, **dadurch gekennzeichnet, daß** die Softwaremodule dynamisch (lernfähig) ausgelegt sind.

40. System nach Anspruch 39, **dadurch gekennzeichnet, daß** die Softwaremodule für eine Parameteranpassung durch "Training" durch den Implantatträger und/oder unter Zuhilfenahme weiterer externer Hilfsmittel adaptiv ausgelegt sind.

41. System nach einem der vorhergehenden Ansprüche, **dadurch gekennzeichnet, daß** die Signalverarbeitungselektronik (42, 44) ein Softwaremodul zum Approximieren einer möglichst optimalen Simulation eines "gesunden" cochleären Verstärkers auf der Basis eines lernfähigen neuronalen Netzwerkes enthält.

42. System nach Anspruch 41, **dadurch gekennzeichnet, daß** das neuronale Netzwerk für ein "Training" durch den Implantatträger und/oder für ein Lernen unter Zuhilfenahme externer Hilfsmittel ausgelegt ist.

43. System nach Anspruch 42, **dadurch gekennzeichnet, daß** in dem neuronalen Netzwerk zur Simulation eines "gesunden" cochleären Verstärkers das Prinzips der "Time-Reversed Acoustics" (TRA) implementiert ist und die Ansteuerung der ausgangsseitigen elektromechanischen Wandler (14, 14', 24) mittels TRA so erfolgt, daß örtlich begrenzte Bereiche der Cochlea mechanisch stimuliert werden.

44. System nach einem der Ansprüche 38 bis 43, **dadurch gekennzeichnet, daß** dem Signalprozessor (42) zur Aufnahme und Wiedergabe eines Betriebsprogramms eine wiederholt beschreibbare, implantierbare Speicheranordnung (S₁, S₂) zugeordnet ist, und mindestens Teile des Betriebsprogramms durch von der externen Einheit (48) über die Telemetrieeinrichtung (46) übermittelte Daten geändert oder ausgetauscht werden können.

45. System nach Anspruch 44, **dadurch gekennzeichnet, daß** ferner eine Zwischenspeicheranordnung (S₄, S₅) vorgesehen ist, in welcher von der externen Einheit (48) über die Telemetrieeinrichtung (46) übermittelte Daten vor dem Weiterleiten an den Signalprozessor (42) zwischengespeichert werden können.

46. System nach Anspruch 45, **dadurch gekennzeichnet, daß** ferner eine Überprü fungslogik (44) vorgesehen ist, um in der Zwischenspeicheranordnung (S₄, S₅) gespeicherte Daten vor dem Weiterleiten an den Signalprozessor (42) einer Überprüfung zu unterziehen.

47. System nach einem der Ansprüche 33 bis 46, **gekennzeichnet durch** einen Mikroprozessorbaustein (44), insbesondere einen Microcontroller, zum implantatinternen Steuern der A/D-Wandler (41) und/oder der D/A-Wandler (43) und/oder des Signalprozessors (42) über einen Datenbus (50).

48. System nach den Ansprüchen 46 und 47, **dadurch gekennzeichnet, daß** die Überprüfungslogik und die Zwischenspeicheranordnung (S₄, S₅) in dem Mikroprozessorbaustein (44) implementiert sind.

49. System nach Anspruch 47 oder 48, **dadurch gekennzeichnet, daß** über den Datenbus (50) und die Telemetrieeinrichtung (46) auch Programmteile oder ganze Softwaremodule zwischen der Außenwelt, dem Mikroprozessorbaustein (44) und dem Signalprozessor (42) übermittelbar sind.

50. System nach einem der Ansprüche 47 bis 49, **dadurch gekennzeichnet, daß** dem Mikroprozessorbaustein (44) eine implantierbare Speicheranordnung (S3) zum Speichern eines Arbeitsprogramms für den Mikroprozessorbaustein zugeordnet ist, und mindestens Teile des Arbeitsprogramms für den Mikroprozessorbaustein durch von der externen Einheit (48) über die Telemetrieeinrichtung (46) übermittelte Daten geändert oder ausgetauscht werden können.

51. System nach einem der Ansprüche 44 bis 50, **dadurch gekennzeichnet, daß** mindestens zwei Speicherbereiche (S₁, S₂) zur Aufnahme und Wiedergabe mindestens des Betriebsprogramms des Signalprozessors (42) vorgesehen sind.

52. System nach einem der Ansprüche 45 bis 51, **dadurch gekennzeichnet, daß** die Zwischenspeicheranordnung mindestens zwei Speicherbereiche (S₄, S₅) zur Aufnahme und Wiedergabe von von der externen Einheit (48) über die Telemetrieeinrichtung (46) übermittelten Daten aufweist.

53. System nach einem der Ansprüche 33 bis 52, **dadurch gekennzeichnet, daß** dem Signalprozessor (42) ferner ein vorprogrammierter, nicht überschreibbarer Fest speicherbereich (S₁) zugeordnet ist.

54. System nach einem der Ansprüche 33 bis 53, **dadurch gekennzeichnet, daß** die Telemetrieeinrichtung (46) zur Übermittlung auch von Betriebsparametern zwischen dem implantierbaren Teil (34) des Systems und der externen Einheit (48) ausgelegt ist.

55. System nach einem der vorhergehenden Ansprüche, **dadurch gekennzeichnet, daß** es vollimplantierbar ausgebildet und mit mindestens einem implantierbaren Schallsensor (40) versehen ist, die elektrische Energieversorgungseinheit implantatseitig ein nachladbares elektrisches Speicherelement (52) aufweist und eine drahtlose, transkutane Ladevorrichtung (55, 56) zum Laden des Speicherelements vorgesehen ist.

56. System nach Anspruch 55, **gekennzeichnet durch** eine drahtlose Fernbedienung (54) zur Steuerung der Implantatfunktionen **durch** den Implantatträger.

57. System nach einem der Ansprüche 1 bis 55, **dadurch gekennzeichnet, daß** es teilimplantierbar ausgebildet ist, wobei mindestens ein Schallsensor (40), die elektronische Anordnung (62) zur Audiosignalverarbeitung und -verstärkung, die Energieversorgungseinheit (52) sowie eine Modulator/Sender-Einheit (63) in einem extern am Körper, vorzugsweise am Kopf über dem Implantat (34'), zu tragenden externen Modul (64) enthalten sind, sowie das Implantat energetisch passiv ist und seine Betriebsenergie und Wandlersteuerdaten über die Modulator/Sender-Einheit im externen Modul empfängt.

58. System nach einem der vorhergehenden Ansprüche, **dadurch gekennzeichnet, daß** es als binaurales System zur Rehabilitation einer Hörstörung beider Ohren ausgelegt ist, das zwei Systemeinheiten aufweist, die jeweils einem der beiden Ohren zugeordnet sind.

59. System nach Anspruch 58, **dadurch gekennzeichnet, daß** die beiden Systemeinheiten einander im wesentlichen gleich sind.

60. System nach Anspruch 58, **dadurch gekennzeichnet, daß** die eine Systemeinheit als Master-Einheit und die andere Systemeinheit als von der Master-Einheit gesteuerte Slave-Einheit ausgelegt ist.

61. System nach einem der Ansprüche 58 bis 60, **gekennzeichnet durch** eine drahtgebundene implantierbare Leitungsverbindung (66), über welche die Signalverarbeitungsmodule (34) so miteinander kommunizieren, daß in beiden Systemeinheiten eine optimierte binaurale Signalverarbeitung und Wandler-Array-Ansteuerung erreicht wird.

62. System nach einem der Ansprüche 58 bis 60, **gekennzeichnet durch** eine drahtlose Verbindung (67), vorzugsweise eine bidirektionale Hochfrequenzstrecke, über welche die Signalverarbeitungsmodule (34) so miteinander kommunizieren, daß in beiden Systemeinheiten eine optimierte binaurale Signalverarbeitung und Wandler-Array-Ansteuerung erreicht wird.

63. System nach einem der Ansprüche 58 bis 60, **dadurch gekennzeichnet, daß** die Signalverarbeitungsmodule (34) unter Verwendung von Ultraschall-Kopplern (69) über eine körperschallgekoppelte Ultraschallstrecke (68) so miteinander kommunizieren, daß in beiden Systemeinheiten eine optimierte binaurale Signalverarbeitung und Wandler-Array-Ansteuerung erreicht wird.

64. System nach einem der Ansprüche 58 bis 60, **dadurch gekennzeichnet, daß** den Signalverarbeitungsmodulen (34) implantierbare Elektroden (72) zugeordnet sind, die im implantierten Zustand Teil einer durch Körpergewebe des Implantatträgers führenden Datenübertragungsstrecke (73) für eine Kommunikation der Signalverarbeitungsmodule (34) der beiden Systemeinheiten sind.

65. System nach einem der vorhergehenden Ansprüche, **dadurch gekennzeichnet, daß** der oder die elektromechanischen Wandler (14') als Hohlkörper ausgebildet sind, die bei Anlegen eines Spannungssignals eine dynamische Volumenänderung erfahren.

66. System nach Anspruch 65, **dadurch gekennzeichnet, daß** der oder die elektromechanischen Wandler (14') in einem schlauchförmigen Träger (11) untergebracht ist (sind), der benachbart von mindestens einem Wandlerende mit mindestens einer Öffnung (82) für den Durchtritt von intracochleärer Flüssigkeit versehen ist.
